# FASCICULE DE BREVET EUROPEEN

(11) **EP 3 092 303 B1**
(45) Date de publication et mention de la délivrance du brevet: **19.08.2020**
(21) Numéro de dépôt: 15700549.7
(22) Date de dépôt: 12.01.2015
(51) Int. Cl.: C12N 5/071, C07K 14/78, C07K 14/52, C07K 14/495, C12N 9/04, G01N 33/50

(54) **MODELE DE PEAU DE MAMMELON RECONSTITUE**
REKONSTITUIERTES MAMILLENHAUTMODELL
RECONSTITUTED NIPPLE SKIN MODEL

(30) Priorité: 10.01.2014 FR 1450193
(43) Date de publication de la demande: 16.11.2016
(73) Titulaire: Laboratoires Expanscience, 92048 Paris La Défense Cedex (FR)
(72) Inventeur: BREDIF, Stéphanie, 28210 Croisilles (FR); BAUDOUIN, Caroline, 78120 Rambouillet (FR); MSIKA, Philippe, 78000 Versailles (FR); MELONI, Marisa, 20145 Milan (IT)
(74) Mandataire: Regimbeau
(86) Numéro de dépôt international: PCT/EP2015/050422
(87) Numéro de publication internationale: WO 2015/104413

(56) Documents cités:
- WO-A1-2013/014435
- MAHLER BRYON ET AL: "Keratin 2e: a marker for murine nipple epidermis", CELLS TISSUES ORGANS, KARGER, BASEL, CH, vol. 176, no. 4, 1 janvier 2004 (2004-01-01), pages 169-177, XP009179291, ISSN: 1422-6405
- JOKE A BOUWSTRA ET AL: "Water Distribution and Natural Moisturizer Factor Content in Human Skin Equivalents Are Regulated by Environmental Relative Humidity", JOURNAL OF INVESTIGATIVE DERMATOLOGY, 2 août 2007 (2007-08-02), XP055131657, ISSN: 0022-202X, DOI: 10.1038/sj.jid.5700994
- C. R. HARDING ET AL: "Filaggrin - revisited", INTERNATIONAL JOURNAL OF COSMETIC SCIENCE, vol. 35, no. 5, 18 octobre 2013 (2013-10-18), pages 412-423, XP055131210, ISSN: 0142-5463, DOI: 10.1111/ics.12049
- MÉHULD ASSELINEAUD BERNARDJ LECLAIREM RÉGNIERR SCHMIDTF BERNERD B: "Gene expression profiles of three different models of reconstructed human epidermis and classical cultures of keratinocytes using cDNA arrays", ARCHIVES OF DERMATOLOGICAL RESEARCH,, vol. 296, no. 4, 1 January 2004 (2004-01-01), pages 145-156, XP009194974,
- Stéphanie Bredif: "Development of an original reconstructed tissue model reproducing nipple epithelium to evaluate anti-inflammatory and repairing efficacy of", , 1 January 2014 (2014-01-01), XP055470069, Retrieved from the Internet: URL:http://www.vitroscreen.com/wp-content/ uploads/2014/05/2014-POSTER-NIPPLE-EPIDERM IS-MODEL_SID.pdf [retrieved on 2018-04-24]

## Description

La présente invention concerne un modèle de peau pouvant avantageusement être utilisé pour étudier les peaux riches en filaggrine et en kératine, telles que par exemple la peau de mamelon ou la peau au niveau des lèvres. La présente invention comprend aussi un procédé pour évaluer l'effet de produits topiques cosmétiques, pharmaceutiques ou dermatologiques sur la peau du mamelon, en particulier sur la cicatrisation de la peau du mamelon et sur la modulation de l'inflammation de la peau du mamelon, de préférence utilisant ledit modèle de peau, ainsi que des kits pour la mise en oeuvre du procédé.

La peau du mamelon peut être sujette à des lésions dermatologiques particulières, appelées crevasses. La crevasse peut être définie d'un point de vue médical comme une ulcération linéaire profonde. Elle est le plus souvent associée à une inflammation.

Ce type de lésions est par exemple retrouvé dans le cas d'eczéma ou de maladie de Paget du sein. Le plus souvent cependant, les crevasses sont observées chez les femmes dans les périodes d'allaitement.

Les crevasses du mamelon sont des lésions apparaissant sous la forme de petites brèches de la peau et situées au niveau des mamelons de la femme. Elles apparaissent essentiellement lors des premiers jours de l'allaitement. Le plus souvent, les crevasses résultent d'un mauvais positionnement du bébé lors des tétées, ou d'une prise de sein incorrecte.

Tandis que les crevasses liées à l'eczéma ou à la maladie de Paget du sein nécessitent un traitement thérapeutique particulier, les crevasses liées à l'allaitement sont bénignes et disparaîtront d'elles-mêmes avec l'arrêt de l'allaitement. Il n'en reste pas moins que ces crevasses peuvent s'accompagner de désagréments tels que des mamelons sensibles, voire irrités, du fait de l'inflammation présente. Des complications sont aussi possibles, telles qu'un léger écoulement de sang lors de l'allaitement, ainsi que, dans des cas plus rares, une surinfection bactérienne.

Restaurer l'intégrité de la peau du mamelon reste la mesure la plus efficace pour prévenir ces désagréments. Nombreux sont donc ceux qui cherchent à développer des produits cosmétiques ou thérapeutiques efficaces qui permettent de soulager rapidement les irritations, faciliter la résorption de ces crevasses et, de cette façon, prévenir toute complication éventuelle.

Cependant, une des étapes limitantes à l'identification et à la caractérisation de ces nouveaux produits est l'évaluation de leur efficacité pour l'utilisation recherchée. Pour permettre le développement rapide de produits cosmétiques ou thérapeutiques sûrs et efficaces, des modèles *in vitro* adaptés et prédictifs sont nécessaires.

De nombreux modèles de peau humaine ont été développés et sont utilisés dans des tests prédictifs de l'activité cosmétique ou thérapeutique d'agents ou de compositions topiques.

Néanmoins, ces modèles ne sont pas adaptés à l'étude de la physiologie et des pathologies de la peau du mamelon. Celle-ci en effet présente des spécificités moléculaires et histologiques qui impactent son fonctionnement.

Le mamelon est un épiderme spécialisé qui possède certaines caractéristiques : hyperkératinisé et épais avec des couches suprabasales, granuleuses et cornifiées étendues. En comparaison avec la peau « normale », par exemple la peau au niveau du ventre, la peau du mamelon est hyperkératinisée avec une surexpression et une distribution spécifique de certains marqueurs tels que la filaggrine et la kératine 14 (Mahler B, Cells Tissues Organs, 176(4):169-77, 2004).

Il a en effet été remarqué chez la souris que la kératine 14, qui est retrouvée dans la partie basale des kératinocytes au niveau de la peau du ventre, est présente dans la partie apicale des kératinocytes au niveau de la peau du mamelon (Panchal et al., BMC Developmental Biology, 7:105, 2007). L'augmentation du niveau de kératine 14, et sa distribution particulière révèlent un tissu dont les cellules prolifèrent et donc qui se renouvèle rapidement. En outre, la filaggrine est un marqueur bien connu qui confère résistance et protection à la peau et ainsi joue un rôle déterminant dans la fonction barrière. L'augmentation du niveau de filaggrine dans la peau du mamelon est ainsi susceptible de jouer un rôle dans sa capacité de cicatrisation.

Malheureusement, aucun modèle se rapprochant de la peau de mamelon n'a été proposé à ce jour.

Il existe toujours un besoin pour un modèle qui reproduise les caractéristiques de la peau du mamelon. Il existe donc un besoin pour des modèles *in vitro* de peau de mamelon, ainsi que des procédés *in vitro* permettant de tester l'activité cosmétique ou thérapeutique d'agents ou de compositions topiques sur cet épiderme particulier.

Les inventeurs ont mis au point un modèle de peau *in vitro* spécialisé permettant de reproduire les caractéristiques biologiques de la peau du mamelon, et pouvant notamment être utilisé pour cribler des actifs ou encore des formulations cosmétiques ou pharmacologiques d'intérêts.

Le modèle a pour particularité de se rapprocher de la véritable composition de la peau du mamelon en ce qu'il comprend une quantité importante des marqueurs biologiques filaggrine et kératine 14. Le modèle comprend en effet des kératinocytes surexprimant de la filaggrine, et avantageusement, de la kératine 14. Ainsi, le modèle de peau comprend des kératinocytes particuliers, qui présentent la particularité d'exprimer de la filaggrine à un niveau supérieur à celui habituellement observé dans des cultures de kératinocytes. Par ailleurs, la production du modèle fait intervenir des facteurs de croissance connus pour stimuler l'expression de kératine 14.

Le modèle de peau est donc particulièrement simple à mettre en oeuvre. En outre, il ne nécessite pas d'utiliser une lignée cellulaire commerciale particulière, et s'avère versatile et adaptable, pourvu que les kératinocytes utilisés présentent les caractéristiques fonctionnelles requises.

Les caractéristiques particulières du modèle permettent d'étudier *in vitro* les particularités de la peau de mamelon. En particulier, il est possible d'étudier les processus de cicatrisation et d'inflammation de la peau de mamelon à partir du modèle *in vitro,* par exemple après avoir induit une lésion sur le modèle.

Il est ici décrit un modèle de peau caractérisé/ spécialisé en ce qu'il comprend des kératinocytes surexprimant la filaggrine.

La présente invention concerne en particulier un modèle de peau de mamelon humaine tel que défini dans les revendications. Le modèle de peau de la description peut être tout modèle tissulaire comprenant des kératinocytes et dans lequel les kératinocytes surexpriment la filaggrine. Ainsi, le modèle de peau peut être tout modèle tissulaire comprenant des kératinocytes. Le modèle de peau n'est donc pas strictement limité à un type de modèle tissulaire particulier, et peut être adapté aux besoins de l'homme du métier.

Par « modèle tissulaire comprenant des kératinocytes», on entend ici toute culture *in vitro* de cellules cutanées comprenant au moins des kératinocytes. Ainsi, les termes « modèle tissulaire comprenant des kératinocytes» comprennent les cultures de kératinocytes en monocouche, les cultures de cellules cutanées en bicouche comprenant des kératinocytes, ainsi que les modèles tissulaires tels que les cultures de peaux reconstruites comprenant des kératinocytes. Avantageusement, le modèle de peau comprend les cultures de peau reconstruite comprenant des kératinocytes.

Les cellules cutanées comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées comprennent au moins des kératinocytes et/ou des fibroblastes.

Les cultures de cellules cutanées en monocouche ou en bicouche sont connues et utilisées depuis très longtemps, et ne nécessitent pas de description détaillée.

Par ailleurs, de nombreux modèles de peaux reconstruites sont à la disposition de l'homme du métier (qui pourra se référer en particulier à Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009 ; Kinicoglu et al., Biomaterials, 32(25) : 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1) : 47-55, 2012; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729 ; WO 2006/063864 ; WO 2006/063865 ; WO 2007/064305).

Par exemple, le modèle de peau reconstruite est sélectionné dans le groupe comprenant les modèles d'épiderme constitués principalement de kératinocytes, les modèles de peau comprenant un derme et un épiderme, et les modèles de peau comprenant un hypoderme, un derme, et un épiderme. Les modèles comprenant au moins un épiderme forment des épithélia stratifiés comprenant les couches caractéristiques du tissu considéré. Par exemple, on peut identifier dans les modèles d'épiderme une couche basale (stratum basale), une couche épineuse (stratum spinosum), une couche granuleuse (stratum granulosum), et une couche cornée (stratum corneum).

Avantageusement, le modèle de peau de l'invention est un modèle d'épiderme comprenant un support matriciel de préférence choisi parmi :
- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable.

Dans ce groupe on trouve les modèles Epiderme reconstruit (Skinethic®) ainsi que le modèle EpiDerm®, (Mattek Corporation) ;
- un film, une membrane ou une matrice à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin® (L'Oréal).

Ces modèles peuvent en outre être ensemencés ou non par des fibroblastes dans la partie dermique.

Avantageusement, sont introduites dans le modèle de peau de la description, des cellules pigmentaires, des cellules immunocompétentes, des cellules nerveuses, de préférence les cellules immunocompétentes sont des cellules de Langerhans. De façon générale, lorsque le modèle de peau de l'invention est un modèle d'épiderme, le support matriciel est produit et ensuite ensemencé par les kératinocytes de l'invention et de la description pour reconstruire l'épiderme et obtenir finalement une peau reconstruite comprenant des kératinocytes capables de surexprimer la filaggrine.

Dans le contexte de l'invention, le terme « kératinocytes » désigne à la fois des kératinocytes primaires et des kératinocytes immortalisés, comme par exemple des kératinocytes provenant de lignées cellulaires. Des lignées de cellules de kératinocytes sont bien connues de l'homme de l'art et comprennent par exemple la lignée de kératinocytes humains HaCaT.

De préférence, les kératinocytes sont d'origine humaine. Par exemple, les kératinocytes proviennent d'échantillons biologiques provenant d'un sujet humain.

De préférence, les kératinocytes utilisés dans le modèle de peau sont obtenus de sujets sains, quelle que soit leur origine.

On entend ici par « sujet sain » un sujet exempt de pathologies cutanées. De préférence, on entend par « sujet sains » des sujets ne présentant pas de lésion cutanée, en particulier ne présentant pas d'atrophie, de bulle, de dyschromie, d'érythème (et exanthème), de kératose, de macule, de nodule, de papule, de purpura, de pustule, de squame, de sclérose, de tumeur, d'ulcération, de condylome, de vésicule.

Les kératinocytes utilisés dans le modèle de peau sont de préférence obtenus d'échantillons biologiques sains provenant d'un sujet humain.

On entend ici par « échantillons biologiques sains » des échantillons biologiques ne présentant pas de lésion cutanée, en particulier ne présentant pas d'atrophie, de bulle, de dyschromie, d'érythème (et exanthème), de kératose, de macule, de nodule, de papule, de purpura, de pustule, de squame, de sclérose, de tumeur, d'ulcération, de condylome, de vésicule.

Les kératinocytes humains peuvent en particulier être isolés à partir d'échantillons de peau, par des techniques de culture cellulaire bien connues de l'homme du métier.

Par exemple, les kératinocytes peuvent être des kératinocytes obtenus à partir d'explant de tissu cutané, en particulier des échantillons d'épithélium malpighien kératinisé. Les kératinocytes humains peuvent ensuite facilement être cultivés *in vitro* selon des techniques bien connues. L'homme du métier pourra en particulier se référer à Leigh et al. (Arch Dermatol Res.;286(1):53-61.1994).

Par « explant » ou « explant de peau », on entend ici un prélèvement de cellules ou de tissu cutané, lequel peut être réalisé dans un but thérapeutique ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède par exemple à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans la présente description.

Par « kératinocytes immortalisés » on entend ici des kératinocytes qui se divisent au-delà de la limite de Hayflick. Ces cellules ont ainsi acquis la capacité de se multiplier indéfiniment, soit suite à une mutation aléatoire ou à une modification délibérée.

Il est bien connu que les cellules normales ne peuvent se diviser qu'un nombre déterminé de fois. Une fois cette limite atteinte, les cellules deviennent sénescentes puis meurent. La limite de Hayflick correspond au nombre de divisions qui peuvent être effectuées par une cellule normale, c'est-à-dire une cellule ne pouvant se diviser qu'un nombre déterminé de fois, avant de cesser de se diviser. Au sens de la présente description, la limite de Hayflick correspond au nombre de divisions qui peuvent être effectuées par un kératinocyte normal avant de cesser de se diviser.

Les kératinocytes peuvent être immortalisés suite à des anomalies particulières, comme c'est le cas par exemple des cellules cancéreuses, ou suite à la mise en oeuvre d'une technique d'immortalisation. Les techniques d'immortalisation sont bien connues de l'homme de l'art qui peut facilement choisir parmi celles-ci la technique la mieux adaptée à son objectif. Par exemple, et sans limiter l'invention à ces exemples, on peut citer comme technique d'immortalisation la transformation par un oncogène, en particulier par l'antigène T de SV40, la protéine Ras, la protéine myc, la protéine Abl. Comme autres techniques on peut citer par exemple la surexpression d'une transcriptase inverse de la télomérase, par exemple hTERT, ou la culture des kératinocytes à confluence de plusieurs passages. Toutes ces techniques sont des techniques de biologie cellulaire classiques qui n'ont pas besoin d'être détaillées ici.

Le marqueur biologique filaggrine comprend le gène FLG humain (référence NCBI : Gene ID: 2312) et les produits de ce gène. Dans un mode de réalisation particulier, le marqueur biologique filaggrine consiste en le gène FLG humain (référence NCBI : Gene ID: 2312). Selon un autre mode de réalisation, le marqueur biologique filaggrine consiste en l'un des produits du gène FLG humain. Les produits du gène FLG humain comprennent le transcrit du gène FLG humain et la protéine filaggrine humaine. Par « transcrit du gène FLG humain » on entend ici le polynucléotide dont la séquence a pour référence NCBI NM_002016.1. Par « protéine filaggrine humaine », on entend ici la protéine dont la séquence peptidique est la séquence de référence NCBI NP_002007.1.

Par « kératinocytes surexprimant la filaggrine », on entend au sens de la présente invention, des kératinocytes présentant de façon constitutive un niveau d'expression du marqueur biologique filaggrine supérieur à un niveau d'expression de référence.

Le niveau d'expression du marqueur biologique filaggrine de référence correspond à un niveau d'expression de kératinocytes de référence, tels que par exemple des kératinocytes primaires issus de la même espèce que les kératinocytes analysés.

Au sens de l'invention, le niveau d'expression du marqueur biologique filaggrine dans les kératinocytes correspond à la quantité des transcrits du gène FLG humain, en particulier du polynucléotide dont la séquence a pour référence NCBI NM_002016.1, dans lesdits kératinocytes. Préférentiellement, le niveau d'expression du marqueur biologique filaggrine correspond au niveau d'expression du polynucléotide dont la séquence a pour référence NCBI NM_002016.1.

N'importe quelle technologie habituellement utilisée par l'homme du métier peut être mise en oeuvre pour mesurer le niveau d'expression du marqueur biologique filaggrine dans les kératinocytes. Comme expliqué plus loin, l'homme du métier pourra en particulier utiliser les procédés d'analyse du niveau d'expression des gènes au niveau nucléotidique, comme par exemple l'analyse du transcriptome. Ces méthodes incluent des méthodes bien connues telles que la RT-PCR ou la RT-PCR quantitative ou encore les puces d'acides nucléiques, et sont détaillés ci-après.

Les kératinocytes surexprimant la filaggrine peuvent aisément être obtenus par l'homme du métier par des techniques classiques de sélection habituellement utilisées en culture cellulaire. De façon générale, à chacun des passages de cellules habituellement réalisés selon les techniques classiques de culture cellulaire, l'homme du métier isolera les kératinocytes dans lesquels le niveau de filaggrine est particulièrement important. Après plusieurs passages, l'homme du métier obtiendra ainsi des kératinocytes surexprimant la filaggrine. Pour plus de détails concernant les méthodes permettant de sélectionner des kératinocytes possédant des caractéristiques particulières, l'homme du métier pourra en particulier se référer à Martin et al. (Eur J Dermatol.;21 Suppl 2:12-20. 2011).

Lors de la production du modèle de peau, il est possible d'utiliser dans le milieu de culture des cellules, des facteurs de croissances capables d'induire la surexpression de la kératine 14. Ce type de facteur de croissance est bien connu de l'homme du métier, et comprend par exemple le sérum foetal de veau (SVF) et le facteur de croissance des fibroblastes b (bFGF). Préférentiellement, le milieu de culture du modèle de peau comprend au moins 5% de sérum foetal de veau, en volume par rapport au volume total du milieu de culture. De cette manière, les kératinocytes du modèle de peau résultant surexpriment la kératine 14.

Préférentiellement, le modèle de peau de la description comprend des kératinocytes surexprimant la kératine 14.

Par « kératinocytes surexprimant la kératine 14», on entend ici, des kératinocytes présentant un niveau d'expression constitutif du marqueur biologique kératine 14 supérieur à un niveau d'expression de référence.

Au sens de la description, le niveau d'expression du marqueur biologique kératine 14 de référence correspond à un niveau d'expression moyen pour des kératinocytes.

Au sens de la description, le niveau d'expression du marqueur biologique kératine 14 dans les kératinocytes correspond à la quantité des transcrits du gène KRT14 humain dont la séquence a pour référence NCBI 3861, en particulier du polynucléotide dont la séquence a pour référence NCBI NM_000526, dans lesdits kératinocytes. Préférentiellement, le niveau d'expression du marqueur biologique kératine 14 correspond au niveau d'expression du polynucléotide dont la séquence a pour référence NCBI NM_002016.1.

Là encore, n'importe quelle technologie habituellement utilisée par l'homme du métier peut être mise en oeuvre pour mesurer le niveau d'expression du marqueur biologique kératine 14 dans le modèle de peau comprenant des kératinocytes.

Le modèle de peau comprend donc des kératinocytes particuliers, qui surexpriment des marqueurs clés, la filaggrine et la kératine 14, qui sont retrouvés dans la peau du mamelon.

Le modèle permet ainsi de reproduire les tissus très riches en filaggrine, tels que la peau du mamelon ou la peau des lèvres chez l'Homme. Ce modèle peut donc être utilisé pour étudier ce type de tissus, ou l'effet de certaines formulations ou de certains actifs sur ce type de tissus. Selon un mode de réalisation, le modèle de l'invention est utilisé pour étudier la peau du mamelon. Ainsi, un objet de l'invention est l'utilisation d'un modèle de peau selon l'invention, pour étudier la peau du mamelon.

En outre, l'homme de l'art peut aussi adapter le modèle pour des usages spécifiques. Par exemple, le modèle peut être modifié de façon à présenter une lésion. Ce type de modèle modifié peut ainsi par exemple être utilisé pour étudier les lésions ou les phénomènes de cicatrisation dans des tissus comme la peau des lèvres ou la peau du mamelon.

Ainsi, le modèle de peau présente une lésion. De préférence, la lésion est une lésion mimant une crevasse. Comme cela est bien connu de l'homme du métier, les crevasses peuvent être définies d'un point de vue médical comme une ulcération linéaire profonde, possiblement associée à une inflammation. Les crevasses apparaissent sous la forme de petites brèches de la peau.

Ainsi, au sens de l'invention, une lésion mimant une crevasse est une lésion reproduisant une ulcération linéaire, de préférence une ulcération linéaire profonde.

De préférence, une lésion mimant une crevasse se présente sous la forme d'une ou de plusieurs brèches du modèle de peau.

Afin d'obtenir un tel modèle, l'homme du métier pourra choisir de provoquer une lésion sur un modèle de peau de l'invention, par exemple de façon mécanique. Par exemple, l'homme du métier pourra provoquer une lésion mécanique profonde simplement en utilisant un objet coupant, tel qu'un scalpel ; ou une lésion superficielle en utilisant tout autre objet non tranchant mais permettant de réaliser une abrasion mécanique de surface. Ce type d'outil permet en effet de reproduire au mieux les crevasses observées *in vivo.*

Les lésions de la peau du mamelon cicatrisent difficilement lors de l'allaitement, et sont en outre très inflammées, et donc très douloureuses. Ainsi, il peut être intéressant de chercher à évaluer l'efficacité de formulations ou encore d'actifs sur la cicatrisation de l'épiderme lésé, ou encore sur la diminution de l'inflammation associée aux lésions. Le modèle de l'invention peut ainsi avantageusement être utilisé dans des procédés d'évaluation de l'activité *in vitro* d'une formulation ou bien d'un actif, en particulier réalisés en vue de tester l'activité de ladite formulation sur la cicatrisation de la peau du mamelon ou sur la diminution de l'inflammation de la peau du mamelon. Le modèle de l'invention peut en outre être utilisé dans des procédés d'identification d'une formulation ou d'un actif adapté à la peau du mamelon lésée, et plus particulièrement permettant de traiter les crevasses du mamelon.

Les inventeurs ont en particulier identifié des marqueurs impliqués dans différents aspects de la cicatrisation ainsi que des marqueurs impliqués dans l'inflammation cutanée.

Ainsi, les marqueurs sélectionnés par les inventeurs permettent d'évaluer l'effet de formulations sur des problématiques différentes mais complémentaires associées aux lésions cutanées. L'utilisation de l'ensemble de ces marqueurs permet donc d'obtenir une évaluation plus complète et exhaustive de l'activité de la formulation ou de l'actif d'intérêt sur la réparation des lésions, mais aussi sur la diminution de l'inflammation de la peau du mamelon pendant ce processus.

Le modèle de peau permet d'observer une variation de la production de ces marqueurs, notamment la lactate déshydrogénase (LDH), le TNFα, la filaggrine, l'intégrine β1 et le TGF β1. Les procédés, en utilisant la mesure du niveau de production d'au moins un de ces marqueurs, permettent donc de suivre l'effet d'une formulation sur la cicatrisation de la peau du mamelon. En fonction des marqueurs biologiques observés, le procédé d'évaluation de l'invention et de la description permet non seulement de déterminer si la formulation stimule la cicatrisation, mais aussi s'il contribue à diminuer l'inflammation présente dans les lésions, en particulier dans les crevasses.

Les formulations et compositions cosmétiques et pharmaceutiques, comprennent un grand nombre de composés, dont certains sont utilisés afin d'obtenir une texture et/ou une viscosité particulière. Ces composés peuvent néanmoins avoir un effet non désiré sur les lésions, par exemple un effet pro-inflammatoire. Au contraire d'autres actifs peuvent présenter des propriétés intéressantes et avoir un effet bénéfique sur les processus de cicatrisation ou sur l'inflammation.

Les méthodes de la description sont aussi adaptées à l'évaluation de l'activité de principes actifs isolés. Les méthodes permettent ainsi de déterminer de façon précise quels actifs ont un effet avantageux sur la cicatrisation de la peau du mamelon.

L'invention a ainsi pour deuxième objet un procédé d'évaluation de l'activité *in vitro* d'un actif ou une formulation sur la cicatrisation de la peau du mamelon et/ou sur la diminution de l'inflammation de la peau du mamelon, caractérisé en ce que ledit procédé comprend au moins les étapes suivantes:
a) mettre en contact ledit actif ou ladite formulation avec un modèle de peau comprenant des kératinocytes surexprimant la filaggrine et comprenant en outre une lésion, tel que décrit plus haut;
b) mesurer le niveau de production d'au moins un marqueur biologique dans le modèle de peau de l'étape a), caractérisé en ce que ledit marqueur biologique est choisi parmi le groupe consistant en:
   - les marqueurs de l'intégrité épidermique, ledit marqueur de l'intégrité épidermique étant de préférence la lactate déshydrogénase (LDH); et
   - les marqueurs de la barrière épidermique, ledit marqueur de la barrière épidermique étant de préférence la filaggrine; et
   - les marqueurs de l'inflammation épidermique, ledit marqueur de l'inflammation épidermique étant de préférence choisi parmi les cytokines, en particulier le TNFα; et
   - les marqueurs de la cicatrisation, ledit marqueur de la cicatrisation étant de préférence choisi parmi les intégrines, en particulier l'intégrine β1, et le TGF β1;
c) comparer le niveau de production d'au moins un marqueur biologique obtenu à l'étape b) avec un niveau de production de référence ;
d) évaluer l'efficacité dudit actif ou de ladite formulation sur la cicatrisation de la peau du mamelon et/ou sur la diminution de l'inflammation de la peau du mamelon en fonction de la comparaison de l'étape c).

Comme cela est bien connu de l'homme du métier, la cicatrisation de la peau correspond à un phénomène biologique naturel de réparation de lésions localisées des tissus. Ce phénomène permet à la peau de recouvrer son intégrité physique après une lésion.

Par exemple, dans un premier temps, l'actif d'intérêt est mis en contact avec un modèle de peau comprenant des kératinocytes surexprimant la filaggrine et comprenant en outre une lésion selon l'invention.

La mise en contact de l'actif d'intérêt avec le modèle de peau de mamelon lésé peut être faite directement, si la formulation de l'actif le permet. Dans certains cas, il pourra être avantageux de formuler l'actif d'intérêt, par exemple de façon à obtenir une composition liquide, afin de faciliter sa mise en contact avec le modèle de peau de mamelon lésé.

Ainsi, par exemple, le procédé comprend en outre une étape de formulation de l'actif, notamment sous forme d'une solution liquide, en particulier aqueuse, préalablement à l'étape de mise en contact dudit actif avec un modèle de peau de mamelon lésé.

Selon un autre exemple, dans un premier temps, la formulation d'intérêt est mise en contact avec un modèle de peau comprenant des kératinocytes surexprimant la filaggrine et comprenant en outre une lésion selon l'invention. La mise en contact de la formulation d'intérêt avec le modèle de peau peut être faite directement.

Après avoir mis en contact l'actif ou la formulation d'intérêt et le modèle de peau, l'homme du métier pourra procéder à la mesure du niveau de production des marqueurs biologiques dans le modèle de peau ainsi traité.

Par « marqueur biologique », on entend ici une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances et de paramètre divers. Par exemple, un marqueur biologique peut être une substance dont la détection indique un état pathologique particulier (par exemple la présence de la protéine C activée en tant que marqueur d'une infection), ou au contraire une substance dont la détection indique un état physiologique spécifique. Le marqueur biologique est préférentiellement un gène, les produits d'un gène tels que ses transcrits et les peptides issus de ses transcrits, un lipide, un sucre ou un métabolite.

Au sens de la présente description, le marqueur biologique est un gène, les produits d'un gène tels que des transcrits ou des peptides, un lipide, un sucre ou un métabolite dont les changements de production, en particulier de niveau de production, corrèlent avec un état physiologique de la peau de de mamelon.

Selon l'invention, le marqueur biologique est choisi parmi les marqueurs de l'intégrité épidermique, les marqueurs de la barrière épidermique, les marqueurs de l'inflammation épidermique.

De préférence, le marqueur de l'intégrité épidermique est la lactate déshydrogénase (LDH).

Selon la description, le marqueur de la barrière épidermique est choisi parmi la filaggrine, les kératines, en particulier la kératine 1 et la kératine 10, l'involucrine, la loricrine, les transglutaminases, les céramides, le cholestérol et les acides gras.

De préférence, le marqueur de la barrière épidermique est la filaggrine.

Selon la description, le marqueur de l'inflammation épidermique est choisi parmi les cytokines, en particulier le TNF α et les interleukines 1, 6 et 8 ; les leukotriènes et les prostaglandines.

De préférence, le marqueur de l'inflammation épidermique est choisi parmi les cytokines, en particulier le TNFα.

Selon la description, le marqueur de la cicatrisation est choisi parmi les intégrines, en particulier l'intégrine β1, l'intégrine β4, l'intégrine α2, l'intégrine α3, l'intégrine α6, la laminine 5, les métalloprotéinases, en particulier les métalloprotéinases MMP1, MMP2 et MMP9, les facteurs de croissance, en particulier le TGF β1 (*Transforming Growth Factor β1*), le KGF (*Keratinocyte Growth Factor*), l'EGF (*Epidermal Growth Factor*), l'interféron gamma.

Le marqueur de la cicatrisation est de préférence choisi parmi les intégrines, en particulier l'intégrine β1, et le TGF β1.

Ces marqueurs ont été sélectionnés par les inventeurs car ils sont spécifiquement associés à l'intégrité de la peau du mamelon.

Dans le processus de cicatrisation, le TNFα est libéré dans la lésion et favorise la migration et l'activation des polynucléaires neutrophiles et des macrophages, qui sont nécessaires à la réparation tissulaire. Cependant, ce marqueur biologique est un facteur de croissance bien connu pour son effet inflammatoire. Or, le signal inflammatoire contribue à la douleur ressentie dans le cas de crevasse au niveau de la peau du mamelon. Ainsi, une baisse du niveau de production du TNFα au niveau des crevasses de la peau de mamelon peut être corrélée à une baisse de l'inflammation, et par conséquent un apaisement des tiraillements dus aux crevasses. Les variations de niveau de production de ce marqueur permettent ainsi d'évaluer l'effet anti-inflammatoire de l'actif d'intérêt au cours du processus de cicatrisation, et donc l'effet apaisant de l'actif sur les crevasses.

Le marqueur biologique TNFα comprend le gène TNFα humain (référence NCBI : Gene ID: 7124), ainsi que les produits de ce gène. Par exemple, le marqueur biologique TNFα consiste en le gène TNFα humain (référence NCBI : Gene ID: 7124). Selon un autre exemple, le marqueur biologique TNFα consiste en l'un des produits du gène TNFα humain. Les produits du gène TNFα humain comprennent le transcrit du gène TNFα humain et la protéine TNFα humaine. Au sens de la description, le transcrit du gène TNFα humain est le polynucléotide dont la séquence a pour référence NCBI : NM_000594. Par protéine TNFα humaine, on entend ici la protéine dont la séquence peptidique est la séquence de référence NCBI : NP_000585.

Par « TNFα sous sa forme peptidique », on entend ici un peptide dont la séquence peptidique comprend la séquence peptidique de la protéine TNFα humaine (référence NCBI : NP_000585).

Par « TNFα sous sa forme nucléotidique », on entend ici au moins un polynucléotide dont la séquence comprend la séquence du transcrit du gène TNFα humain (référence NCBI : Gene ID: 7124). Au sens de la présente description, le transcrit du gène TNFα humain est le polypeptide dont la séquence a pour référence NCBI : NM_000594.

La LDH est une enzyme qui est normalement présente dans le cytosol des cellules. Sa présence dans le milieu de cellules en culture témoigne d'un dommage cellulaire. Ainsi, l'augmentation du relargage de ce marqueur est corrélée à une augmentation des dommages cellulaires, c'est-à-dire des lésions. Au contraire, la diminution du relargage de ce marqueur témoigne d'une diminution des dommages cellulaires, et donc des lésions. La diminution du niveau de production de ce marqueur est donc corrélée à une évolution positive du processus de cicatrisation. Les variations de niveau de production de ce marqueur permettent ainsi d'évaluer l'effet de l'actif d'intérêt sur le processus de cicatrisation, et son effet protecteur et réparateur. La lactate déshydrogénase (LDH) est une enzyme tétramérique. Chez les mammifères, chaque sous-unité peut être soit de type H (anglais heart = cœur) ou M (muscle). Il existe plusieurs isotypes de LDH qui diffèrent suivant le type d'assemblage formé par ces sous-unités. La sous-unité M est codée par le gène LDHA (référence NCBI : Gene ID: 3939), tandis que la sous-unité H est codée par le gène LDHB (référence NCBI : Gene ID: 3945). La forme prédominante dans la peau est la forme LDH-2, qui comprend trois sous-unité H et une sous unité M.

Le marqueur biologique lactate déshydrogénase (LDH) est préférablement le marqueur LDH-2, qui comprend trois sous-unité H, et une sous unité M.

La filaggrine est un marqueur retrouvé de façon importante dans les cellules de la peau du mamelon, c'est d'autre part un marqueur de la barrière épidermique. Lors du processus de cicatrisation, la réparation des tissus s'accompagne d'une prolifération et d'une différenciation des cellules, venant combler la lésion afin de restaurer l'épiderme, et donc d'une augmentation du taux de production de la filaggrine. L'augmentation du niveau de production de ce marqueur est donc corrélée à une évolution positive du processus de cicatrisation, et donc à la réparation du tissu lésé avec restauration d'un épiderme intègre. Les variations de niveau de production de ce marqueur permettent ainsi d'évaluer l'effet de l'actif d'intérêt sur le processus de cicatrisation, et son effet réparateur.

Par « filaggrine sous sa forme peptidique », on entend ici un peptide dont la séquence peptidique comprend la séquence peptidique de la protéine filaggrine humaine (référence NCBI : NP_002007.1).

Par « filaggrine sous sa forme nucléotidique », on entend préférablement ici au moins un polynucléotide dont la séquence comprend la séquence du transcrit du gène FLG humain (référence NCBI : Gene ID: 2312). Au sens de la présente description, le transcrit du gène FLG humain est le polynucléotide dont la séquence a pour référence NCBI NM_002016.1.

L'intégrine β1 est un facteur de croissance qui contrôle de nombreuses fonctions cellulaires, impliqué dans la régulation de la cicatrisation. L'augmentation du niveau de production de ce marqueur indique une amplification du processus de cicatrisation et de la réparation du tissu lésé. Les variations de niveau de production de ce marqueur permettent ainsi d'évaluer l'effet de l'actif d'intérêt sur le processus de cicatrisation, et son effet réparateur.

Au sens de la présente description, le marqueur biologique intégrine β1 comprend le gène ITGB1 humain (référence NCBI : Gene ID: 3688), ainsi que les produits de ce gène. Par exemple, le marqueur biologique intégrine β1 consiste en le gène ITGB1 humain (référence NCBI : Gene ID: 3688). Selon un autre exemple, le marqueur biologique intégrine β1 consiste en l'un des produits du gène ITGB1 humain. Les produits du gène ITGB1 humain comprennent les transcrits du gène ITGB1 humain et les protéines intégrine β1 humaine. Les transcrits du gène ITGB1 humain comprennent les polynucléotides dont la séquence est choisie parmi les séquences de référence NCBI NM_002211.3, NM_133376.2 and NM_033668.2. Par protéine intégrine β1 humaine, on entend icila protéine dont la séquence peptidique est choisie parmi les séquences de référence NCBI: NP_002202.2, NP_596867.1 et NP_391988.1.

Par « intégrine β1 sous sa forme peptidique », on entend préférablement un peptide dont la séquence peptidique est choisie parmi les séquences de référence NCBI : NP_002202.2, NP_596867.1 et NP_391988.1.

Par « intégrine β1 sous sa forme nucléotidique », on entend préférablement au moins un polynucléotide dont la séquence comprend la séquence du transcrit du gène ITGB1 humain (référence NCBI : Gene ID: 3688). Au sens de la présente invention et de la présente description, les transcrits du gène ITGB1 humain comprennent les polynucléotides dont la séquence est choisie parmi les séquences de référence NCBI NM_002211.3, NM_133376.2 et NM_033668.2.

Le TGF β1 (pour « *Transforming Growth factor beta 1* », ou Facteur de croissance transformant bêta 1) est un polypeptide membre de la famille des facteurs de croissance transformant bêta, qui appartient elle-même à la superfamille des cytokines. Ce peptide est en particulier impliqué dans le contrôle de la croissance et de la prolifération cellulaire. Au niveau de la peau, et dans le cadre du phénomène de cicatrisation, une augmentation du niveau de production de TGF β1 est corrélée à la réparation de l'épiderme. L'augmentation du niveau de production de ce marqueur indique une amplification du processus de cicatrisation et de la réparation du tissu lésé. Les variations de niveau de production de ce marqueur permettent ainsi d'évaluer l'effet de l'actif d'intérêt sur le processus de cicatrisation, et son effet réparateur.

Le marqueur biologique TGF β1 comprend le gène TGFB1 humain (référence NCBI : Gene ID: 7040), ainsi que les produits de ce gène. Par exemple, le marqueur biologique TGF β1 consiste en le gène TGFB1 humain (référence NCBI : Gene ID: 7040). Selon un autre exemple, le marqueur biologique TGF β1 consiste en l'un des produits du gène TGFB1 humain. Les produits du gène TGFB1 humain comprennent le transcrit du gène FLG humain et le polypeptide TGF β1 humain. Au sens de la présente description, le transcrit du gène TGFB1 humain est le polynucléotide dont la séquence a pour référence NCBI NM_000660. Par polypeptide TGF β1 humain, on entend préférablement le polypeptide dont la séquence peptidique est la séquence de référence NCBI : NP_000651.

Par « TGF β1 sous sa forme peptidique », on entend préférablement un peptide dont la séquence peptidique est la séquence de référence NCBI : NP_000651.

Par « TGF β1 sous sa forme nucléotidique », on entend préférablement au moins un polynucléotide dont la séquence comprend la séquence du transcrit du gène TGFB1 humain (référence NCBI : Gene ID: 7040). Au sens de la présente description, le transcrit du gène TGFB1 humain est le polynucléotide dont la séquence est la séquence de référence NCBI NM_000660.

L'homme de l'art comprendra qu'il est possible de sélectionner par exemple des combinaisons de marqueurs d'un type particulier. En utilisant un seul type de marqueur, l'homme du métier peut obtenir une réponse précise sur l'efficacité de la formulation ou de l'actif, telle que par exemple une évaluation en particulier de l'efficacité sur l'inflammation épidermique ou une évaluation en particulier de l'efficacité sur la cicatrisation. L'homme du métier pourrait ainsi utiliser par exemple des combinaisons de marqueurs consistant en des marqueurs de l'intégrité épidermique, des combinaisons de marqueurs consistant en des marqueurs de la barrière épidermique, des combinaisons de marqueurs consistant en des marqueurs de l'inflammation épidermique, des combinaisons de marqueurs consistant en des marqueurs de la cicatrisation.

Il sera par ailleurs évident à l'homme du métier que la méthode de la description permettra une évaluation de l'efficacité de la formulation ou de l'actif d'autant plus complète qu'un grand nombre de marqueurs de types différents seront utilisés. L'homme du métier pourra par exemple évaluer à la fois l'efficacité de la formulation ou de l'actif sur l'inflammation épidermique et sur la cicatrisation.

D'autre type de combinaisons de marqueurs sont aussi envisageables, comme par exemple des combinaisons de deux types de marqueurs, telles que :
- des combinaisons de marqueurs consistant en des marqueurs de l'intégrité épidermique et des marqueurs de la barrière épidermique, en particulier la combinaison de marqueurs consistant en la lactate déshydrogénase (LDH) et la filaggrine ;
- des combinaisons de marqueurs consistant en des marqueurs de l'intégrité épidermique et des marqueurs de l'inflammation épidermique, en particulier la combinaison de marqueurs consistant en la lactate déshydrogénase (LDH) et le TNFα ;
- des combinaisons de marqueurs consistant en des marqueurs de l'intégrité épidermique et des marqueurs de la cicatrisation, en particulier la combinaison de marqueurs consistant en la lactate déshydrogénase (LDH) et l'intégrine β1 ou la combinaison de marqueurs consistant en la lactate déshydrogénase (LDH) et le TGF β1;
- des combinaisons de marqueurs consistant en des marqueurs de la barrière épidermique et des marqueurs de l'inflammation épidermique, en particulier la combinaison de marqueurs consistant en la filaggrine et le TNFα;
- des combinaisons de marqueurs consistant en des marqueurs de la barrière épidermique et des marqueurs de la cicatrisation, en particulier la combinaison de marqueurs consistant en la filaggrine et l'intégrine β1 ou la combinaison de marqueurs consistant en la filaggrine et le TGF β1; ou
- des combinaisons de marqueurs consistant en des marqueurs de l'inflammation épidermique et des marqueurs de la cicatrisation, en particulier la combinaison de marqueurs consistant en le TNFα et l'intégrine β1 ou la combinaison de marqueurs consistant en le TNFα et le TGF β1.

Avantageusement, l'homme du métier pourra utiliser des combinaisons de trois types de marqueurs, telles que :
- des combinaisons de marqueurs consistant en des marqueurs de l'intégrité épidermique, des marqueurs de la barrière épidermique et des marqueurs de l'inflammation épidermique, en en particulier la combinaison de marqueurs consistant en la lactate déshydrogénase (LDH), la filaggrine et le TNFα ;
- des combinaisons de marqueurs consistant en des marqueurs de l'intégrité épidermique, des marqueurs de la barrière épidermique et des marqueurs de la cicatrisation, en particulier la combinaison de marqueurs consistant en la lactate déshydrogénase (LDH), la filaggrine et le TGF β1, ou la combinaison de marqueurs consistant en la lactate déshydrogénase (LDH), la filaggrine et l'intégrine β1;
- des combinaisons de marqueurs consistant en des marqueurs de l'intégrité épidermique, des marqueurs de l'inflammation épidermique et des marqueurs de la cicatrisation, en particulier la combinaison de marqueurs consistant en la lactate déshydrogénase (LDH), le TNFα et le TGF β1, ou la combinaison de marqueurs consistant en la lactate déshydrogénase (LDH), le TNFα et l'intégrine β1 ;
- des combinaisons de marqueurs consistant en des marqueurs de la barrière épidermique, des marqueurs de l'inflammation épidermique et des marqueurs de la cicatrisation, en particulier la combinaison de marqueurs consistant en la filaggrine, le TNFα et le TGF β1, ou la combinaison de marqueurs consistant en la filaggrine, le TNFα et l'intégrine β1.

De préférence, l'étape b) comprend la mesure du niveau de production d'une combinaison de marqueurs biologiques consistant en la lactate déshydrogénase (LDH), le TNFα, la filaggrine, l'intégrine β1 et le TGF β1.

Ainsi, le marqueur biologique peut être un gène, ou le produit d'un gène présentant une activité particulière, telle qu'une activité enzymatique par exemple.

Pour chacun de ces types de marqueurs, de nombreuses méthodes sont à la disposition de l'homme du métier pour mesurer le niveau de production dudit marqueur biologique. Selon le type de marqueur utilisé, les termes « niveau de production « peuvent se référer à des variables différentes, qui dépendent de la nature du marqueur examiné.

Par exemple, lorsque le marqueur biologique est un gène, les termes « niveau de production du marqueur biologique » se réfèrent généralement au niveau de synthèse d'au moins un des produits de ce gène. Plus précisément, lorsque le marqueur biologique est un gène, le niveau de production dudit marqueur biologique correspond à la quantité ou à la concentration d'au moins un des transcrits dudit gène ou d'au moins un des différents isoformes des protéines issus desdits transcrits.

Lorsque le marqueur est le produit d'un gène présentant une activité enzymatique, les termes « niveau de production du marqueur biologique » se réfèrent préférentiellement au niveau d'activité enzymatique dudit marqueur. Au sens de la présente description, l'activité enzymatique dudit marqueur correspond à la quantité de substrat dudit marqueur catalysée par unité de temps grâce à un quantum défini dudit marqueur. L'unité d'activité enzymatique U est classiquement exprimée en µmοl/min ou en kat (mol/s). L'unité d'activité enzymatique U peut aussi être exprimée en mg/min lorsque le substrat de l'enzyme existe dans le corps sous forme de polymère, tel que c'est le cas par exemple lorsque le substrat est choisi parmi les sucres ou les glycosaminoglucanes.

L'homme du métier cherchant à déterminer si un des produits du gène d'intérêt présente une activité enzymatique, pourra facilement consulter la littérature scientifique pertinente ou se rapporter à des bases de données publiques telles que, par exemple, celles regroupées sur le site internet ExPASy, qui répertorie notamment les enzymes (http://enzyme.expasy.org).

De préférence, le marqueur biologique LDH est le produit d'un gène présentant une activité enzymatique.

Pour mesurer le niveau d'activité enzymatique d'un marqueur, l'homme du métier pourra utiliser toute technique bien connue dans l'art. En particulier, la mesure de l'activité enzymatique de l'enzyme LDH peut être réalisée par un test enzymatique: la LDH relarguée réduit le NAD+ en NADH+H+ par oxydation du lactate en pyruvate, dans une seconde étape, un catalyste (diaphorase) transfère 2 H du NADH+H+ au sel de tetrazolium INT qui est alors réduit en formazan. La formation de formazan est révélée par l'induction d'une coloration mesurée en colorimétrie. La quantité de formazan formée est directement corrélée à l'activité enzymatique de la LDH dans le surnageant. Par exemple, la mesure de l'activité enzymatique de l'enzyme LDH peut être réalisée au moyen d'un test colorimétrique basé sur la détection de sels de formazan à l'aide d'un kit commercial (par exemple *cytotoxicity detection kit-LDH*, Roche).

Par la « mesure du niveau de production d'une combinaison de marqueurs biologiques », on entend ici la mesure du niveau de production de chacun des marqueurs de la combinaison. La production d'un gène peut être mesurée par exemple au niveau nucléotidique, en mesurant la quantité de transcrits dudit gène et leurs variants, et peut aussi être mesurée par exemple au niveau peptidique, en mesurant par exemple la quantité d'un ou plusieurs isoformes des protéines issus desdits transcrits, et leurs variants. Ainsi, par « mesure du niveau de production dudit gène » on entend au sens de l'invention la mesure de la quantité de produit du gène sous sa forme peptidique ou sous sa forme nucléotidique.

Dans ce cas, le procédé peut comprendre une ou plusieurs étapes préalable(s) à la mesure de la production du marqueur biologique, lesdites étapes correspondant à l'extraction à partir du modèle de peau de mamelon de l'étape a), d'un échantillon d'ARNm (ou de l'ADNc correspondant) ou d'un échantillon de protéine. Celui-ci peut ensuite être directement utilisé pour mesurer la production du marqueur. La préparation ou l'extraction d'ARNm (ainsi que la rétrotranscription de celui-ci en ADNc) ou de protéines à partir d'un tissu tel qu'un modèle de peau ou de cellules ne sont que des procédures de routine bien connues de l'homme du métier.

De préférence, le niveau de production du marqueur choisi parmi la LDH, le TNFα, le TGFβ1 et la filaggrine, correspond au niveau de production dudit marqueur sous sa forme nucléotidique. De préférence, le niveau de production du marqueur intégrine β1 correspond au niveau de production dudit marqueur sous sa forme peptidique.

Pour chacun des types de marqueurs biologiques, de nombreux procédés sont à la disposition de l'homme du métier pour mesurer le niveau de production dudit marqueur biologique.

Quand le marqueur biologique est un gène, et que le niveau de production du marqueur est mesuré au niveau nucléotidique, c'est-à-dire en mesurant la quantité de produit du gène sous sa forme nucléotidique, n'importe quelle technologie habituellement utilisée par l'homme du métier peut être mise en oeuvre. Les procédés d'analyse du niveau de production des gènes au niveau nucléotidique, comme par exemple l'analyse du transcriptome, incluent des procédés bien connus tels que la RT-PCR ou la RT-PCR quantitative ou encore les puces d'acides nucléiques. Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose. Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides. Pour déterminer le profil de production d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Préférentiellement, la méthode est mise en oeuvre en utilisant toute méthode actuelle ou future permettant de déterminer la production des gènes sur la base de la quantité d'ARNm dans l'échantillon. Par exemple, l'homme du métier peut mesurer la production d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de la production des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente *in situ.* Pour l'analyse au niveau de l'ARNm, les puces à tissu peuvent être couplées avec l'hybridation fluorescente in situ. Enfin, il est possible d'utiliser le séquençage massif en parallèle pour déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676- 684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

En outre, toute méthode pour déterminer le niveau de production d'un polypeptide connue de l'homme du métier peut être utilisée. Les méthodes pour déterminer le niveau de production d'un polypeptide incluent par exemple la spectrométrie de masse, les tests biochimiques, y compris les tests immunologiques tels que par exemple les tests immunologiques de détection classiques (les tests ELISA et les tests ELISPOTS), ou encore par exemple des tests immunologiques employant des techniques de transfert des protéines sur support, tels que le slot blot (aussi appelé dot blot) ou le western blot. Il est par exemple possible d'employer les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques de voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Par «un niveau de production de référence d'un marqueur biologique », on entend ici tout niveau de production dudit marqueur utilisé à titre de référence. Par exemple, un niveau de production de référence peut être obtenu en mesurant le niveau de production du marqueur d'intérêt dans un modèle de peau de mamelon dans des conditions particulières. L'homme du métier saura choisir ces conditions particulières de façon à servir son objectif dans la mise en oeuvre de la méthode.

Ainsi, de préférence, le niveau de production de référence d'un marqueur biologique est le niveau de production dudit marqueur obtenu dans un modèle de peau de mamelon lésé non traité avec la formulation d'intérêt.

Selon un autre exemple, le niveau de production de référence d'un marqueur biologique est le niveau de production dudit marqueur obtenu dans un modèle de peau de mamelon mis en contact avec une formulation de référence.

Préférentiellement, la formulation de référence est choisie parmi la liste constituée des formulations classiquement utilisés en dermatologie ou cosmétique, en particulier des formulations comprenant des principes tels que les agents anti-inflammatoires/anti-irritants ou les agents cicatrisants et restructurants de la barrière cutanée.

Les agents anti-inflammatoires/anti-irritants utilisés en dermatologie et en cosmétique sont bien connus de l'homme du métier. De façon générale, ils limitent la réaction inflammatoire conduite via les cytokines ou médiateurs du métabolisme de l'acide arachidonique et ont des propriétés apaisantes et anti-irritantes. Les agents anti-inflammatoires/anti-irritants pouvant être compris dans une formulation de référence au sens de la description sont l'acide glycyrrhétinique (les dérivés de réglisse) avec ses sels et esters, l'acide lipoïque, le beta-carotène, la vitamine B3 (niacinamide, nicotinamide), la vitamine E, la vitamine C, la vitamine B12, les flavonoïdes (thé vert, quercétine...), le lycopène ou la lutéine, les sucres d'Avocat, l'oléodistillat d'Avocat, l'arabinogalactane, les peptides de Lupin, un extrait total de Lupin, un extrait peptidique de Quinoa, le Cyclocéramide (dérivé d'oxazoline), les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (Lycium barbarum), les peptides ou complexes d'acides aminés végétaux ou encore la disulone topique, ou les médicaments anti-inflammatoires stéroïdiens (AIS), tels que les corticoïdes, ou non-stéroïdiens (AINS).

Les agents cicatrisants et restructurants de la barrière cutanée utilisés en dermatologie et en cosmétique sont bien connus de l'homme du métier. De façon générale, ils stimulent la synthèse des lipides clés de l'épiderme. Les agents cicatrisants et restructurants de la barrière cutanée pouvant être compris dans une formulation de référence au sens de la description sont la vitamine A, le panthénol (vitamine B5), les sucres d'Avocat, l'oléodistillat de Tournesol, le Lupéol, l'extrait peptidique de Maca, un extrait peptidique de Quinoa, l'arabinogalactane, l'oxyde de zinc, le magnésium, le silicium, l'acide madécassique ou asiatique, le sulfate de dextran, le coenzyme Q10, la glucosamine et ses dérivés, la chondroïtine sulfate et globalement les glucosaminoglycanes ou GAG, le sulfate de dextran, les céramides, le cholestérol, le squalane, les phospholipides, les peptides de soja fermenté ou non, les peptides végétaux, les polysaccharides marins, végétaux ou biotechnologiques comme les extraits d'algues ou l'extrait de fougère, les oligo-éléments, les extraits de plantes à tanin comme les tanins dérivant de l'acide gallique appelés tanins galliques ou encore hydrolysables, initialement trouvés dans la noix de galle, et les tanins catéchiques résultant de la polymérisation d'unités flavaniques dont le modèle est fourni par le Cachou (Acacia catechu). Les oligo-éléments pouvant être utilisés sont avantageusement choisis dans le groupe constitué par le cuivre, le magnésium, le manganèse, le chrome, le sélénium, le silicium, le zinc et leurs mélanges. Peuvent également être utilisés des concentrats de Tournesol, plus avantageusement des concentrats de Tournesol linoléiques, tels que l'actif commercialisé par les Laboratoires Expanscience, Soline, des insaponifiables d'huile végétale, tel que l'Avocadofurane®, ou des agonistes PPARs (rosiglitazone, pioglitazone), RXR, LXR.

L'homme du métier pourra en outre utiliser comme formulation de référence toute formulation connue de l'art antérieur, et notamment toute formulation connue pour ses propriétés cicatrisantes et/ou anti-inflammatoires.

A titre d'exemple, on peut citer les compositions suivantes :
- les sucres d'Avocat tels que décrits dans la demande WO 2005/115421. Cette composition est particulièrement adaptée pour le traitement de la réparation de la barrière cutanée et de l'inflammation ;
- les peptides d'Avocat tels que décrits dans la demande WO2005/105123. Cette composition est en effet particulièrement adaptée pour le traitement de l'irritation et de l'inflammation ;
- l'huile d'Avocat telle que décrite dans les demandes WO2004/012496, WO2004/012752, WO2004/016106, WO2007/057439) ;
- les furanes d'avocat, tels que pouvant être obtenus par le procédé décrit dans la demande WO 01/21605. Cette composition, aussi commercialisée sous la marque Avocadofurane® est particulièrement adaptée pour le traitement de l'inflammation, et pour favoriser la cicatrisation;
- le 5 alpha Avocuta® (butyl avocadate);
- les insaponifiables d'Avocat et de Soja. Les insaponifiables d'Avocat et de Soja pouvant être utilisés en association sont avantageusement un mélange d'insaponifiables d'Avocat furaniques et d'insaponifiables de Soja, dans un rapport respectif d'environ 1/3-2/3. Les insaponifiables d'Avocat et de Soja sont encore plus avantageusement le produit Piasclédine®, commercialisé par les Laboratoires Expanscience;
- l'oléodistillat de Tournesol, encore plus avantageusement avec des concentrats de Tournesol linoléiques, tels que l'actif commercialisé par les Laboratoires Expanscience, Soline® (décrit en particulier dans la demande WO 01/21150). Cette composition est particulièrement adaptée pour le traitement de l'inflammation et pour la réparation de la barrière cutanée.
- l'insaponifiable de Soja, tel qu'obtenu selon le procédé décrit dans la demande internationale WO01 /51596;
- le Lupéol (FR 2 822 821, FR 2 857 596). Cette composition est particulièrement adaptée pour favoriser la cicatrisation;
- les peptides de Lupin tels qu'obtenus selon le procédé décrit dans la demande WO2005/102259. Cette composition est particulièrement adaptée pour le traitement de l'inflammation;
- l'extrait total de Lupin (tel que décrit dans la demande WO2005/102259). Cette composition est particulièrement adaptée pour le traitement des irritations;
- l'huile de Lupin, avantageusement une huile de Lupin blanc doux, telle que celle décrite dans la demande internationale WO 98/47479;
- l'extrait peptidique de Maca (en particulier tel que décrit dans la demande WO2004/112742). Cette composition est particulièrement appréciée pour ses propriétés cicatrisantes;
- les peptides de Riz (voir demande WO 2008/009709);
- le Cyclocéramide® (dérivé d'oxazoline) tel que décrit dans les demandes WO2004050052, WO2004050079, et WO2004112741. Cette composition est particulièrement adaptée pour le traitement des réactions inflammatoires ;
- l'extrait de Quinoa, en particulier l'extrait peptidique décrit dans la demande WO2008/080974. Cette composition est particulièrement adaptée pour le traitement des conditions inflammatoires et de la réparation de la barrière cutanée.
- Le beurre de Cupuaçu. Cette composition est particulièrement appréciée pour ses propriétés hydratantes.

Enfin, l'homme du métier pourra utiliser comme formulation de référence toute formulation connue de l'art antérieur pour son effet sur la peau du mamelon, tel que par exemple un baume dont la composition peut être telle que décrite plus spécifiquement dans la partie expérimentale de la présente demande. D'autres formulations de référence bien connues dans le domaine des crevasses de la peau du mamelon sont le lait maternel, dont l'application sur les crevasses est recommandée pour faciliter leur cicatrisation, la glycerine et la lanoline.

L'homme du métier comprendra en outre aisément que la comparaison de l'étape c) est de préférence effectuée entre des mesures de niveaux de production obtenus pour des modèles de peau de mamelon de compositions histologiques similaires, voire identiques. Par « compositions histologiques similaires », on entend ici que les proportions relatives des types cellulaires compris dans les modèles de peau de mamelon comparés sont similaires. Ainsi, il est préférable que les proportions relatives des types cellulaires compris dans le modèle de peau de mamelon de l'étape a) ne diffèrent pas de plus de 5% des proportions relatives des types cellulaires compris dans le modèle de peau de mamelon utilisé pour l'obtention du niveau de production de référence de l'étape c). Par « proportion relative d'un type cellulaire » on entend ici le rapport du nombre de cellules correspondant à ce type cellulaire sur le nombre de cellules totales comprises dans le modèle de peau. Ainsi, il est par exemple préférable que la proportion de kératinocytes sur le nombre de cellules totales dans le modèle de peau de mamelon de l'étape a) ne diffère pas de plus de 5% de la proportion de kératinocytes sur le nombre de cellules totales dans le modèle de peau de mamelon utilisé pour l'obtention du niveau de production de référence de l'étape c). Par « compositions histologiques identiques », on entend ici que les proportions relatives des types cellulaires compris dans les modèles de peau de mamelon comparés sont identiques. Au sens de la présente description, les proportions relatives des types cellulaires compris dans le modèle de peau de mamelon de l'étape a) sont identiques aux proportions relatives des types cellulaires compris dans le modèle de peau de mamelon utilisé pour l'obtention du niveau de production de référence de l'étape c) lorsqu'elles ne diffèrent pas de plus de 0,1%. Avantageusement, la proportion de kératinocytes sur le nombre de cellules totales dans le modèle de peau de mamelon de l'étape a) ne diffère pas de plus de 0,1% de la proportion de kératinocytes sur le nombre de cellules totales dans le modèle de peau de mamelon utilisé pour l'obtention du niveau de production de référence de l'étape c).

L'homme du métier comprendra tout aussi aisément que la comparaison de l'étape c) est de préférence effectuée entre des mesures de niveaux de production obtenus pour des modèles de peau de mamelon qui sont de taille, de volume ou de poids similaires, voire identiques. Ainsi, il est préférable que la taille, le volume, ou le poids du modèle de peau de mamelon de l'étape a) ne diffère pas de plus de 5% de la taille, et/ou du volume et/ou du poids du modèle de peau de mamelon utilisé pour l'obtention du niveau de production de référence de l'étape c). Ainsi, il est préférable que la taille, le volume, ou le poids du modèle de peau de mamelon de l'étape a) ne diffère pas de plus de 5% de la taille, du volume, ou du poids du modèle de peau de mamelon utilisé pour l'obtention du niveau de production de référence de l'étape c). Plus préférentiellement, la taille, et le volume et le poids du modèle de peau de l'étape a) ne diffère pas de plus de 5% de la taille, du volume et du poids du modèle de peau de mamelon utilisé pour l'obtention du niveau de production de référence de l'étape c). Encore plus préférentiellement, la taille, et le volume et le poids du modèle de peau de l'étape a) ne diffère pas de plus de 0.1% de la taille, du volume et du poids du modèle de peau de mamelon utilisé pour l'obtention du niveau de production de référence de l'étape c).

Alternativement, si les modèles de peau diffèrent de plus de 5 % de par la taille, le volume et le poids, l'homme du métier pourra normaliser le niveau obtenu à l'étape b) et le niveau de référence de l'étape c) à l'aide d'un facteur de normalisation.

Ce facteur de normalisation pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau de production d'un gène qui est exprimé au même niveau dans toutes les cellules, ou presque, de l'organisme. En d'autres termes, on utilise par exemple comme facteur de normalisation le niveau de production d'un gène de ménage. Selon un autre mode de réalisation, le niveau obtenu à l'étape b) et le niveau de référence de l'étape c sont normalisés en utilisant le niveau de production, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genetics 19: 362-365, 2003). Les gènes de ménage incluent par exemple les gènes B2M, TFRC, YWHAZ, RPLO, 18S, GUSB, UBC, TBP, GAPDH, PPIA, POLR2A, ACTB, PGK1, HPRT1, IP08 et HMBS.

L'homme du métier pourra ainsi aisément évaluer l'efficacité de la formulation d'intérêt en fonction de la comparaison de l'étape c).

Par exemple, lorsque le niveau de production de LDH mesuré à l'étape b), est inférieur ou égal au niveau de production de ces marqueurs obtenu dans un modèle de peau de mamelon lésé non traité avec la formulation d'intérêt, alors la formulation est efficace sur la cicatrisation de la peau du mamelon.

Par exemple, lorsque le niveau de production de TNFα mesuré à l'étape b), est inférieur ou égal au niveau de production de ces marqueurs obtenu dans un modèle de peau de mamelon lésé non traité avec la formulation d'intérêt, alors la formulation est efficace sur l'inflammation de la peau du mamelon.

Selon un autre exemple, lorsque le niveau de production d'intégrine β1, mesuré à l'étape b), est supérieur ou égal au niveau de production de ces marqueurs obtenu dans un modèle de peau de mamelon lésé non traité avec la formulation d'intérêt, alors la formulation est efficace sur la cicatrisation et/ ou sur l'inflammation de la peau du mamelon.

Selon un autre exemple, lorsque le niveau de production de filaggrine, mesuré à l'étape b), est supérieur ou égal au niveau de production de ces marqueurs obtenu dans un modèle de peau de mamelon lésé non traité avec la formulation d'intérêt, alors la formulation est efficace sur la cicatrisation et/ ou sur l'inflammation de la peau du mamelon.

Selon un autre exemple, lorsque le niveau de production de TGF β1, mesuré à l'étape b), est supérieur ou égal au niveau de production de ces marqueurs obtenu dans un modèle de peau de mamelon lésé non traité avec la formulation d'intérêt, alors la formulation est efficace sur la cicatrisation et/ ou sur l'inflammation de la peau du mamelon.

Selon un autre exemple, lorsque le niveau de production de LDH mesuré à l'étape b), est inférieur ou égal au niveau de production de ces marqueurs obtenu dans un modèle de peau de mamelon mis en contact avec une formulation de référence, alors la formulation d'intérêt est plus efficace sur la cicatrisation de la peau du mamelon que la formulation de référence.

Selon un autre exemple, lorsque le niveau de production de TNFα mesuré à l'étape b), est inférieur ou égal au niveau de production de ces marqueurs obtenu dans un modèle de peau de mamelon mis en contact avec une formulation de référence, alors la formulation d'intérêt est plus efficace sur la cicatrisation de la peau du mamelon que la formulation de référence.

Selon un autre exemple, lorsque le niveau de production d'intégrine β1, mesuré à l'étape b), est supérieur ou égal au niveau de production de ces marqueurs obtenu dans un modèle de peau de mamelon mis en contact avec la une formulation de référence, alors la formulation d'intérêt est plus efficace sur la cicatrisation de la peau du mamelon que la formulation de référence.

Selon un autre exemple, lorsque le niveau de production de filaggrine, mesuré à l'étape b), est supérieur ou égal au niveau de production de ces marqueurs obtenu dans un modèle de peau de mamelon mis en contact avec la une formulation de référence, alors la formulation d'intérêt est plus efficace sur la cicatrisation de la peau du mamelon que la formulation de référence.

Selon un autre exemple, lorsque le niveau de production de TGF β1, mesuré à l'étape b), est supérieur ou égal au niveau de production de ces marqueurs obtenu dans un modèle de peau de mamelon mis en contact avec la une formulation de référence, alors la formulation d'intérêt est plus efficace sur la cicatrisation de la peau du mamelon que la formulation de référence.

Dans un autre aspect, le modèle permet d'isoler des actifs ou des formulations ayant un effet sur la peau de mamelon et, plus particulièrement, lorsque celle-ci est lésée. Le modèle permet donc d'identifier des actifs ou des formulations pour traiter les lésions de la peau du mamelon, en particulier les crevasses.

L'invention a donc également pour objet un procédé d'identification d'un actif ou d'une formulation pour traiter les lésions de la peau du mamelon, en particulier les crevasses, ledit procédé comprenant les étapes suivantes :
a) mettre en contact d'un actif candidat ou d'une formulation candidate avec un modèle de peau comprenant des kératinocytes surexprimant la filaggrine et comprenant en outre une lésion tel que décrit plus haut;
b) mesurer le niveau de production d'au moins un marqueur biologique dans le modèle de peau de mamelon de l'étape a), caractérisé en ce que ledit marqueur biologique est choisi parmi le groupe consistant en :
   - les marqueurs de l'intégrité épidermique, ledit marqueur de l'intégrité épidermique étant de préférence la lactate déshydrogénase (LDH); et
   - les marqueurs de la barrière épidermique, ledit marqueur de la barrière épidermique étant de préférence la filaggrine; et
   - les marqueurs de l'inflammation épidermique, ledit marqueur de l'inflammation épidermique étant de préférence choisi parmi les cytokines, en particulier le TNFα; et
   - les marqueurs de la cicatrisation, ledit marqueur de la cicatrisation étant de préférence choisi parmi les intégrines, en particulier l'intégrine β1, et le TGF β1;
c) comparer le niveau de production d'au moins un marqueur biologique obtenu à l'étape b) avec un niveau de production de référence ;
d) déterminer si ledit actif candidat ou ladite formulation candidate est une formulation pour traiter les lésions de la peau du mamelon, en particulier les crevasses, en fonction de la comparaison de l'étape c).

L'actif candidat ou la formulation candidate est un actif ou une formulation pour traiter les lésions de la peau du mamelon, en particulier les crevasses, si ledit actif ou ladite formulation candidate permet de moduler la production d'au moins un marqueur biologique. Cette modulation peut correspondre, selon les cas, et en particulier selon la nature du marqueur biologique, à une augmentation ou à une diminution de la production dudit marqueur. En particulier, il peut être intéressant d'isoler des actifs ou des formulations stimulant la production des marqueurs corrélés à une amélioration de la cicatrisation ou encore des formulations diminuant l'inflammation.

La la description permet ainsi d'identifier des matières premières améliorant la cicatrisation et diminuant l'inflammation. L'identification de marqueurs biologiques permet par conséquent d'identifier les matières premières modulant ou non la production de ces marqueurs.

Dans un autre aspect, la description permet l'isolement de matière première pouvant être utilisée dans la mise au point de formulations adaptées à la peau de mamelon lésé.

Il est donc ici décrit un procédé d'identification d'une matière première pouvant être utilisée pour la préparation d'une formulation pour traiter les lésions de la peau du mamelon, en particulier les crevasses, ledit procédé comprenant les étapes suivantes :
a) mettre en contact une matière première candidate avec un modèle de peau comprenant des kératinocytes surexprimant la filaggrine et comprenant en outre une lésion tel que décrit plus haut;
b) mesurer le niveau de production d'au moins un marqueur biologique dans le modèle de peau de mamelon de l'étape a), caractérisé en ce que ledit marqueur biologique est choisi parmi le groupe consistant en :
   - les marqueurs de l'intégrité épidermique, ledit marqueur de l'intégrité épidermique étant de préférence la lactate déshydrogénase (LDH); et
   - les marqueurs de la barrière épidermique, ledit marqueur de la barrière épidermique étant de préférence la filaggrine; et
   - les marqueurs de l'inflammation épidermique, ledit marqueur de l'inflammation épidermique étant de préférence choisi parmi les cytokines, en particulier le TNFα; et
   - les marqueurs de la cicatrisation, ledit marqueur de la cicatrisation étant de préférence choisi parmi les intégrines, en particulier l'intégrine β1, et le TGF β1;
c) comparer le niveau de production d'au moins un marqueur biologique obtenu à l'étape b) avec un niveau de production de référence ;
d) déterminer si ladite matière première candidate est une matière première pour la préparation d'une formulation pour traiter les lésions de la peau du mamelon, en particulier les crevasses, en fonction de la comparaison de l'étape c).

De préférence, l'étape b) comprend la mesure du niveau de production d'une combinaison de marqueurs biologiques consistant en la lactate déshydrogénase (LDH), le TNFα, la filaggrine, et l'intégrine β1 et/ou le TGF β1. En d'autres termes, l'étape b) comprend la mesure du niveau de production de chacun des marqueurs biologiques d'une combinaison de marqueurs biologiques consistant en la lactate déshydrogénase (LDH), le TNFα, la filaggrine, et l'intégrine β1 et/ou le TGF β1.

Il est clair que la description permet en outre d'évaluer l'innocuité de certains actifs ou de certaines formulations, ou bien d'identifier parmi ces actifs ou ces formulations ceux ou celles qui présentent des qualités d'innocuité optimales vis-à-vis de la peau de mamelon lésée. On considère que des actifs ou des formulations présentent des qualités d'innocuité si elles ne nuisent pas à la santé humaine lorsqu'elles sont appliquées dans les conditions normales ou raisonnablement prévisibles d'utilisation. L'homme du métier comprendra en effet aisément qu'il ne suffit que de mesurer la production d'un ou plusieurs marqueurs biologiques selon l'invention pour déterminer si une formulation peut être utilisée sur la peau de mamelon lésée.

Il également ici décrit un kit pour la mise en oeuvre d'une méthode la description, comprenant les moyens nécessaires à la mesure du niveau de production d'au moins un marqueur biologique choisi parmi le groupe consistant en:
- les marqueurs de l'intégrité épidermique, ledit marqueur de l'intégrité épidermique étant de préférence la lactate déshydrogénase (LDH); et
- les marqueurs de la barrière épidermique, ledit marqueur de la barrière épidermique étant de préférence la filaggrine; et
- les marqueurs de l'inflammation épidermique, ledit marqueur de l'inflammation épidermique étant de préférence choisi parmi les cytokines, en particulier le TNFα; et
- les marqueurs de la cicatrisation, ledit marqueur de la cicatrisation étant de préférence choisi parmi les intégrines, en particulier l'intégrine β1, et le TGF β1.

En particulier, le kit comprend les moyens nécessaires à la mesure du niveau de production de chacun des marqueurs biologiques de la combinaison consistant en la LDH, le TNFα, la filaggrine, l'intégrine β1 et /ou le TGF β1.

Préférentiellement, les moyens nécessaires à la mesure du niveau de production de l'intégrine β1 sont des anticorps spécifiques desdits marqueurs.

Préférentiellement, les moyens nécessaires à la mesure du niveau de production du TNFα, de la filaggrine, et du TGF β1 comprennent des sondes nucléiques et/ou des amorces d'amplification capables de se lier à au moins l'un de ces marqueurs biologiques sous sa forme nucléotidique.

Les exemples suivants sont fournis ici à titre d'illustration et ne sont pas, sauf indication contraire, destinés à être limitatifs.

### Légendes des figures

Figure 1 : Histologie d'un épiderme reconstruit (à J17) obtenu à partir du lot de kératinocytes choisi pour la mise en place du modèle de mamelon
Figure 2 : Expression génique de la filaggrine dans des peaux reconstruites après 24 heures d'incubation
Figure 3 : Relargage de LDH dans le milieu de culture 4 heures et 24 heures après lésion des peaux reconstruites « mamelon »
Figure 4 : Expression génique du TNFα dans des peaux reconstruites « mamelon » 4 heures après lésion
Figure 5 : Expression génique de filaggrine dans des peaux reconstruites « mamelon » 24 heures après lésion
Figure 6 : Analyse de la production de l'intégrine β1 en Western Blot dans des peaux reconstruites « mamelon » 24 heures après lésion
Figure 7 : Relargage de LDH dans le milieu de culture 4 heures après lésion des épidermes reconstruits « mamelon »
Figure 8 : Expression génique du TNFα dans des épidermes reconstruits « mamelon » 24 heures après lésion
Figure 9 : Expression génique du TGF β1 dans des épidermes reconstruits « mamelon » 24 heures après lésion
Figure 10 : Marquage de l'intégrine β1 dans des épidermes reconstruits « mamelon » 4 heures après lésion (bleu = noyaux, dapi ; rouge = intégrine)
Figure 11 : Visualisation en Microscopie Electronique à Balayage de la surface des épidermes reconstruits « de mamelon » 4 heures après lésion (G x1000)

### Exemples

### Exemple 1 : Développement d'un modèle tissulaire de mamelon pour l'évaluation d'un baume allaitement

Objectifs : Développement d'un modèle mimant les caractéristiques et la morphologie de l'épithélium du mamelon pour mettre en place un modèle expérimental mimant des fissures et crevasses afin d'évaluer l'efficacité de produits cosmétiques comme traitement apaisant (anti-inflammatoire) et réparateur.

Le mamelon est un épiderme spécialisé qui possède certaines caractéristiques : hyperkératinisé et épais avec des couches suprabasales, granuleuses et cornifiées étendues.

Au niveau moléculaire, on note une production accrue et une distribution spécifique de certains marqueurs (fort niveau de production dans de multiples couches de l'épiderme) : filaggrine (marqueur de la barrière → réponse tissulaire différente à des stimuli externes) et kératine 14 (marqueur mitotique, indicatif d'un état hyperprolifératif du tissu).

Le modèle de mamelon a été obtenu par reconstruction d'un épiderme ou d'une peau totale (épiderme reconstruit sur une matrice de collagène) à partir d'un lot de kératinocytes surexprimant la filaggrine.

### A. Sélection du lot de kératinocytes à utiliser pour produire le modèle de mamelon

Des épidermes reconstruits ont été produits à partir de différents lots de kératinocytes et analysés comparativement en histochimie et immunohistochimie.

Le lot choisi pour produire le modèle de mamelon est un lot de kératinocytes surexprimant la filaggrine et, après reconstruction d'un épiderme, présentant un nombre important de couches granuleuses avec de nombreux granules de kératohyaline et un marquage important de filaggrine.

Ce lot de kératinocytes a été utilisé dans les étapes suivantes pour produire des modèles tissulaires mimant le mamelon.

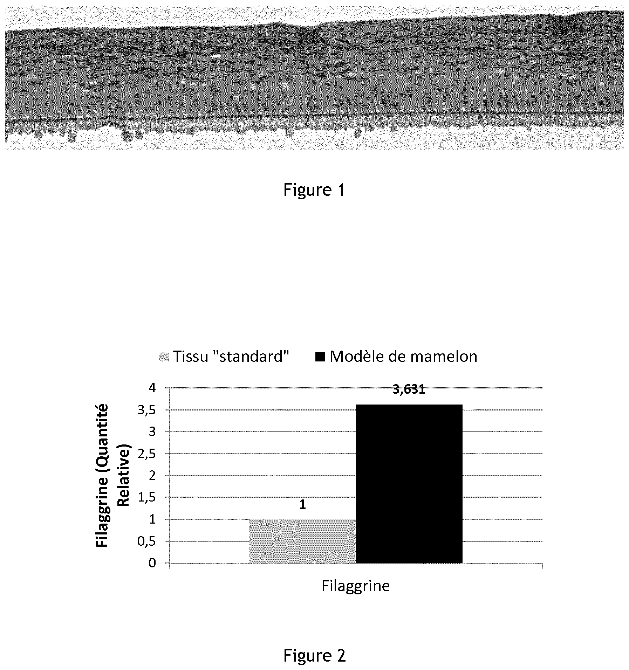

Une coupe d'un épiderme reconstruit (à J17) obtenu à partir du lot de kératinocytes choisi pour la mise en place du modèle de mamelon est illustré figure 1.

### B. Caractérisation d'un modèle de mamelon

### 1. Matériel et Méthodes

Le modèle de mamelon a été obtenu par reconstruction d'une peau (à partir du lot de kératinocytes défini précédemment), sur une matrice de collagène contenant des fibroblastes.

En parallèle, une peau « standard » a été reconstruite dans les mêmes conditions, sur une matrice de collagène contenant des fibroblastes, à partir d'un lot de kératinocytes standard (ne surexprimant pas la filaggrine). Ce tissu standard sert de référence au modèle de tissu de mamelon.

Les tissus reconstruits ont été incubés pendant 4 heures et 24 heures puis l'expression génique de la filaggrine a été étudiée par PCR quantitative (qPCR) en temps réel.

Les peaux reconstruites ont été lysées et les ARN totaux extraits puis rétro-transcrits en cDNA. L'expression du gène d'intérêt (Filaggrine) a été analysée par qPCR en temps réel et comparée à l'expression du gène de ménage GAPDH.

### 2. Résultats

Le modèle de peau de mamelon exprime beaucoup plus de filaggrine qu'un modèle de peau standard (+260%). Ces résultats sont illustrés par la figure 2. Ils confirment que le modèle mis en place se rapproche de la physiologie du mamelon en termes de production de filaggrine.

### C. Etude 1 : Utilisation d'un modèle de peau reconstruite de mamelon pour évaluer l'efficacité réparatrice et anti-inflammatoire d'un baume allaitement

### 1. Matériel et Méthodes

Le modèle de mamelon a été obtenu par reconstruction d'une peau à partir d'un lot de kératinocytes surexprimant la filaggrine (comme décrit précédemment), sur une matrice de collagène contenant des fibroblastes.

Une lésion (blessure mécanique) en surface de la peau reconstruite a été réalisée, afin de mimer une crevasse ou fissure du mamelon. Le produit à l'essai (baume allaitement) a été appliqué topiquement immédiatement après réalisation de la lésion. N'importe quelle formulation de baume peut être testé par la méthode de l'invention et de la description, telles que par exemples les formulations 1 à 3 ci-dessous.

Les résultats présentés ci-après correspondent à ceux obtenus pour le baume dont la composition correspond à la formulation n°3 (« baume allaitement 3 »).

### BAUME ALLAITEMENT 1

| **Matière première / Nom commercial** | **%** |
|---|---|
| EAU PURIFIEE | De 5 à 20% |
| AMIDON | De 5 à 20% |
| GLYCEROL | QSP 100% |

### BAUME ALLAITEMENT 2

| **Matière première / Nom commercial** | **%** |
|---|---|
| BEURRE ou HUILE | De 5 à 20% |
| LANOLINE | QSP 100% |

### BAUME ALLAITEMENT 3

| **Matière première / Nom commercial** | **%** |
|---|---|
| EAU PURIFIEE | QSP 100% |
| BEURRE DE KARITE | De 5 à 20% |
| GLYCEROL | De 5 à 20% |
| DECYL PENTANOATE | De 5 à 20% |
| GELIFIANT DE PHASSE GRASSE | De 1 à 5% |
| METHYLGLUCOSEDIOLATE | De 1 à 5% |
| SYSTEME CONSERVATEUR | De 1 à 5% |
| CIRE VEGETALE | De 1 à 5% |
| ACTIFS | De 1 à 5% |
| EMULSIONNANT | De 1 à 5% |
| GOMME XANTHANE | De 0 à 1% |
| AJUSTEUR pH | De 0 à 1% |
| ANTIOXYDANT | De 0 à 1% |

Les peaux reconstruites ont alors été incubées pendant 4 heures et 24 heures puis les paramètres suivant ont été analysés :
- Relargage de LDH,
- Expression génique (qPCR en temps réel) du TNFα et de la filaggrine,
- Production d'intégrine β1 (Western Blot).

### a. Quantification de la LDH relarguée :

La LDH relarguée dans le milieu de culture des peaux reconstruites a été quantifiée au moyen d'un test colorimétrique basé sur la détection de sels de formazan dont la formation est liée à l'activité enzymatique de l'enzyme. La quantité de LDH a été déterminée au moyen d'une courbe standard et est exprimée en mU/ml.

### b. qPCR en temps réel :

Les peaux reconstruites ont été lysées et les ARN totaux extraits puis rétro-transcrits en cDNA. L'expression des gènes d'intérêt (TNFα, filaggrine) a été analysée par qPCR en temps réel et comparée à l'expression du gène de ménage GAPDH.

### c. Analyse de la production d'intégrine β1 par Western blot :

Les peaux reconstruites ont été lysées, en présence d'inhibiteurs de protéases. Les protéines issues des échantillons ont été quantifiées, séparées par électrophorèse sur gel de polyacrylamide puis transférées sur membrane de nitrocellulose. Les membranes ont alors été incubées en présence d'un anticorps primaire anti-Intégrine β1 (Santa Cruz), puis en présence d'un anticorps secondaire marqué (Invitrogen). Le signal de chemiluminescence a alors été visualisé et quantifié, son intensité est proportionnelle à la quantité d'intégrine β1 produite.

### 2. Résultats

### a. Evaluation de l'intégrité des membranes cellulaires : dosage de LDH

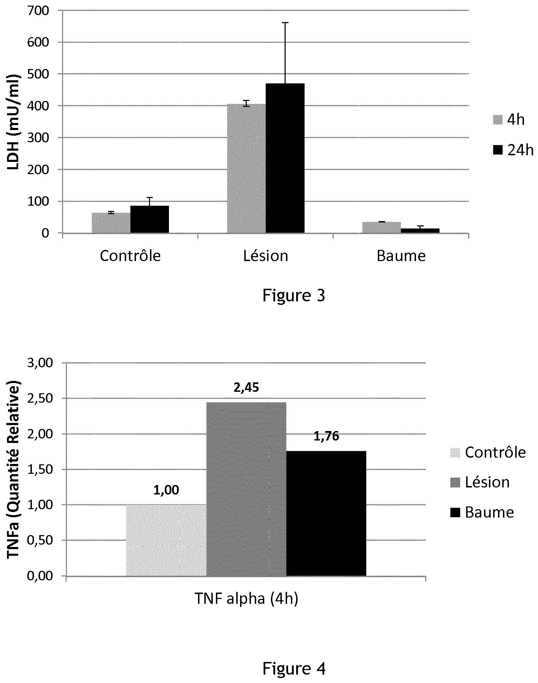

Les résultats du dosage de la LDH sont indiqués dans le tableau 1 et illustrés par la figure 3.

**Tableau 1**

| | LDH (mU/ml) | | | |
|---|---|---|---|---|
| | 4 heures | | 24 heures | |
| Tissu contrôle | 64 ± 2,99 | | 86,5 ± 24,74 | |
| Tissu lésé | 407 ± 9 | +536% | 470,5 ± 191,62 | +444% |
| Tissu lésé + baume | 35 ± 0,86 | -91% | 14,5 ± 7,78 | -97% |

Les membranes cellulaires forment une membrane fonctionnelle autour des cellules. Lorsque les cellules sont endommagées, les membranes sont altérées et deviennent poreuses, perméables à certains composants.

L'intégrité des membranes a été évaluée par mesure de la LDH (lactate deshydrogénase) dans le milieu extracellulaire. Cette enzyme est normalement présente dans le cytosol et est indétectable dans l'espace extracellulaire en l'absence de dommages cellulaires.

La lésion des peaux reconstruites a induit un relargage important de LDH dans l'espace extracellulaire, témoignant de l'altération de l'intégrité des membranes cellulaires. L'application topique du baume allaitement a réduit très fortement le relargage de LDH. Le baume allaitement protège l'intégrité des tissus et accélère leur récupération après la lésion.

### b. Evaluation de la réponse inflammatoire : expression de TNFα

Les résultats de la mesure de l'expression de TNFα sont indiqués dans le tableau 2 et illustrés par la figure 4.

**Tableau 2**

| | TNFα | |
|---|---|---|
| | (Expression génique en quantité relative) | |
| Tissu contrôle | 1,00 | |
| Tissu lésé | 2,45 | +145% |
| Tissu lésé + baume | 1,76 | -28% |

Le (Tumor Necrosis Factor-alpha) est un médiateur important de l'inflammation ; le signal inflammatoire contribue à l'amplification de la douleur ressentie. La lésion des peaux reconstruites a induit une forte augmentation (+145%) de l'expression génique du TNFα, représentative de l'induction d'un signal inflammatoire.

L'application topique du baume allaitement a modulé la surexpression du TNFα induite par la lésion (-28%).

Le baume allaitement module l'inflammation et contribue donc à minimiser la douleur.

### c. Evaluation de la réparation de la barrière : expression de la filaggrine

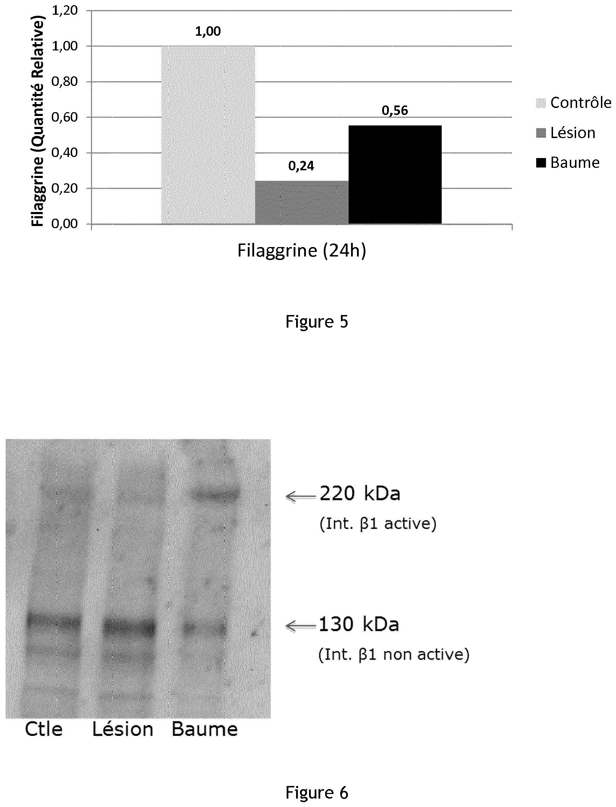

Les résultats de la mesure de la production de la filaggrine sont indiqués dans le tableau 3 et illustrés par la figure 5.

**Tableau 3**

| | Filaggrine | |
|---|---|---|
| | (Expression génique en quantité relative) | |
| Tissu contrôle | 1,00 | |
| Tissu lésé | 0,24 | -76% |
| Tissu lésé + baume | 0,56 | +133% |

La filaggrine a un double rôle essentiel à la fonction barrière : d'une part, elle s'agrège aux fibres de kératine du cytosquelette, réduisant ainsi les cornéocytes en disques aplatis, ce réseau intracellulaire confère résistance et protection au stratum corneum. D'autre part, la filaggrine est le précurseur du NMF (Natural Moisturizing Factor ou Facteur Naturel d'Hydratation). Le NMF est hautement hygroscopique et est essentiel à la rétention d'eau dans les cornéocytes, il joue de plus un rôle majeur dans le maintien du pH de la peau, régulant ainsi des événements biochimiques clés comme l'activité des protéases, la perméabilité de la barrière et les défenses antimicrobiennes, fonctions qui sont fondamentalement liées et co-régulées. La lésion des peaux reconstruites a entrainé une forte diminution (-76%) de l'expression génique de la filaggrine, montrant une altération importante de la fonction barrière.

L'application du baume allaitement a modulé la répression de la filaggrine (+133%). Le baume allaitement tend à contrer l'effet délétère de la lésion sur la barrière et contribue ainsi à accélérer la réparation de la barrière.

### d. Evaluation de l'initiation de la cicatrisation : expression de l'intégrine-β1

Les résultats de la mesure de la production de l'intégrine-β1 sont illustrés par la figure 6.

Les intégrines, un groupe de protéines membranaires, sont des facteurs importants dans la régulation de la cicatrisation. En effet, d'une part, les intégrines permettent la migration des kératinocytes, étape précoce du processus de cicatrisation ; et d'autre part, elles se lient à des macromolécules de la matrice dermique, assurant ainsi le contact de l'épiderme avec la matrice extra-cellulaire dermique nouvellement synthétisée pour permettre la cohésion et la communication entre les deux compartiments (derme et épiderme).

La sous-unité d'intégrine β1 est une protéine transmembranaire de 130 kDa qui forme des complexes non covalents avec différentes sous-unités α pour former des récepteurs fonctionnels et actifs.

Ces récepteurs coordonnent l'activation de voies de signalisation intracellulaire impliquées notamment dans la cicatrisation.

L'analyse en western blot de la production de l'intégrine β1 (figure 6) montre que :
- dans les tissus contrôles et lésés, une seule bande de 130 kDa est observée, cette bande correspond à la sous-unité β1 seule de l'intégrine ;
- en présence du baume allaitement, la sous-unité β1 seule (bande de 130 kDa) diminue et des aggrégats de plus haut poids moléculaire (220 kDa) se sont formés ; ces aggrégats correspondent à la formation de complexes (sous-unité β1 liée à une sous-unité a) ou récepteurs hétérodimériques fonctionnels représentatifs de l'activation de l'intégrine β1.

Le baume allaitement rend fonctionnel l'intégrine β1, et permet ainsi d'initier le processus de cicatrisation.

### 3. Conclusion

Un modèle de mamelon obtenu par reconstruction d'une peau à partir d'un lot de kératinocytes surexprimant la filaggrine a été utilisé afin d'évaluer l'efficacité réparatrice et anti-inflammatoire d'un baume allaitement après lésion du mamelon mimant une crevasse.

Sur ce modèle de mamelon lésé, le baume allaitement à un effet protecteur (protection de l'intégrité des membranes cellulaires / LDH), réparateur (induction de la cicatrisation / intégrine β1 ; restauration de la barrière / filaggrine) et apaisant (anti-inflammatoire / TNFα).

Ces résultats confirment l'intérêt du modèle mis en place comme un modèle mimant la peau de mamelon et permettant d'évaluer l'efficacité réparatrice et apaisante d'une formulation dédiée au soin du mamelon, en particulier dans le cas de l'apparition de crevasses ou fissures.

D. Etude 2 : Utilisation d'un modèle d'épiderme reconstruit de mamelon pour évaluer comparativement l'efficacité réparatrice et anti-inflammatoire de produits cosmétiques pour l'allaitement en comparaison avec le lait maternel

### 1. Matériel et Méthodes

Pour mimer le mamelon, un épiderme reconstruit, obtenu à partir d'un lot de kératinocytes surexprimant la filaggrine (comme décrit précédemment), a été utilisé.

Deux produits cosmétiques destinés à soigner les crevasses liées à l'allaitement ont été étudiés : un baume allaitement (correspondant à la formulation « baume allaitement 3 ») et une pommade à base de lanoline (produit 2). En parallèle, du lait maternel, décrit pour ses propriétés réparatrices a également été évalué.

Une lésion (blessure mécanique) en surface de l'épiderme de mamelon a été réalisée, afin de mimer une crevasse ou fissure du mamelon.

Les produits à l'essai ont ensuite été appliqués topiquement immédiatement après réalisation de la lésion.

Les épidermes ont alors été incubés pendant 4 heures et 24 heures puis les paramètres suivant ont été analysés :
- Relargage de LDH ;
- Expression génique (qPCR en temps réel) du TGF β1 et du TNFα;
- Marquage en immunofluorescence de l'intégrine β1 (uniquement produit 1).

### a. Quantification de la LDH relarguée :

La LDH relarguée dans le milieu de culture des épidermes reconstruits a été quantifiée au moyen d'un test colorimétrique basé sur la détection de sels de formazan dont la formation est liée à l'activité enzymatique de l'enzyme. La quantité de LDH a été déterminée au moyen d'une courbe standard et est exprimée en mU/ml.

### b. qPCR en temps réel :

Les épidermes reconstruits ont été lysés et les ARN totaux extraits puis rétro-transcrits en cDNA. L'expression des gènes d'intérêt (TNFα, TGF β1) a été analysée par qPCR en temps réel et comparée à l'expression du gène de ménage GAPDH.

### c. Marquage en immunofluorescence de l'intégrine β1 (évalué uniquement pour le produit 1) :

Les épidermes reconstruits ont été fixés, enrobés en paraffine et coupés. Les sections ont été incubées en présence d'un anticorps primaire dirigé contre le domaine extracellulaire de l'intégrine β1 (Santa Cruz), puis en présence d'un anticorps secondaire conjugué au fluorochrome Alexa Fluor 555 (Invitrogen). Les noyaux ont été contre-colorés au DAPI.

Les marquages ont été visualisés en microscopie confocale (Leica).

### 2. Résultats

### a. Evaluation de l'intégrité des membranes cellulaires : dosage de LDH

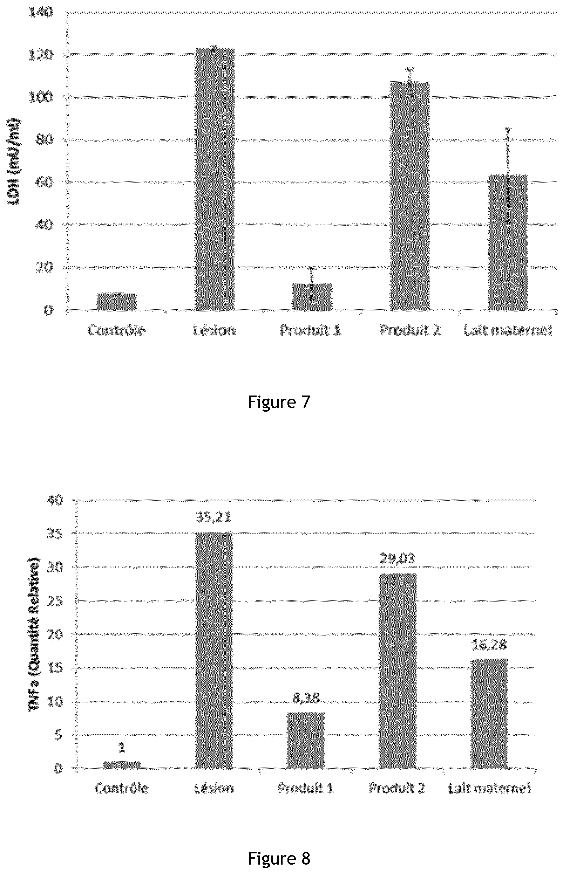

Les résultats sont représentés dans le tableau 4 et illustrés dans la figure 7.

**Tableau 4**

| | LDH (mU/ml) | |
|---|---|---|
| Tissu contrôle | 7,8 ± 0 | |
| Tissu lésé | 123,11 ± 0,86 | |
| Tissu lésé + produit 1 | 12,45 ± 6,98 | -90% |
| Tissu lésé + produit 2 | 106,85 ± 6,06 | -13% |
| Tissu lésé + lait maternel | 63,15 ± 21,94 | -49% |

La lésion des épidermes reconstruits « de mamelon » a induit un relargage important de LDH dans l'espace extracellulaire, représentatif de l'altération de l'intégrité des membranes cellulaires.

L'application topique du produit 1 a réduit très fortement le relargage de LDH (90% d'inhibition). Le produit 2 testé a faiblement diminué le relargage de LDH (-13%), et le lait maternel a entrainé une diminution intermédiaire du relargage de LDH (-49%).

Le produit 1 et le lait maternel, dans une moindre mesure, protègent l'intégrité des tissus et accélèrent leur récupération après la lésion.

### b. Evaluation de la réponse inflammatoire : expression du TNFα

Les résultats sont représentés dans le tableau 5 et illustrés dans la figure 8.

**Tableau 5**

| | TNFα | |
|---|---|---|
| | (Expression génique en quantité relative) | |
| Tissu contrôle | 1,00 | |
| Tissu lésé | 35,21 | |
| Tissu lésé + produit 1 | 8,38 | -76% |
| Tissu lésé + produit 2 | 29,03 | -17% |
| Tissu lésé + lait maternel | 16,28 | -54% |

La lésion des épidermes reconstruits a induit une forte augmentation de l'expression génique du TNFα, représentative de l'induction d'un signal inflammatoire.

Le produit 1 a très nettement inhibé la surexpression du TNFα induite par la lésion (-76%) ; le produit 2 a induit une faible diminution (-17%) et le lait maternel a induit une diminution d'un niveau intermédiaire (-54%).

Le produit 1 et, dans une moindre mesure, le lait maternel modulent l'inflammation et contribuent donc à apaiser la douleur.

### c. Evaluation de l'initiation de la cicatrisation : expression du TGF β1

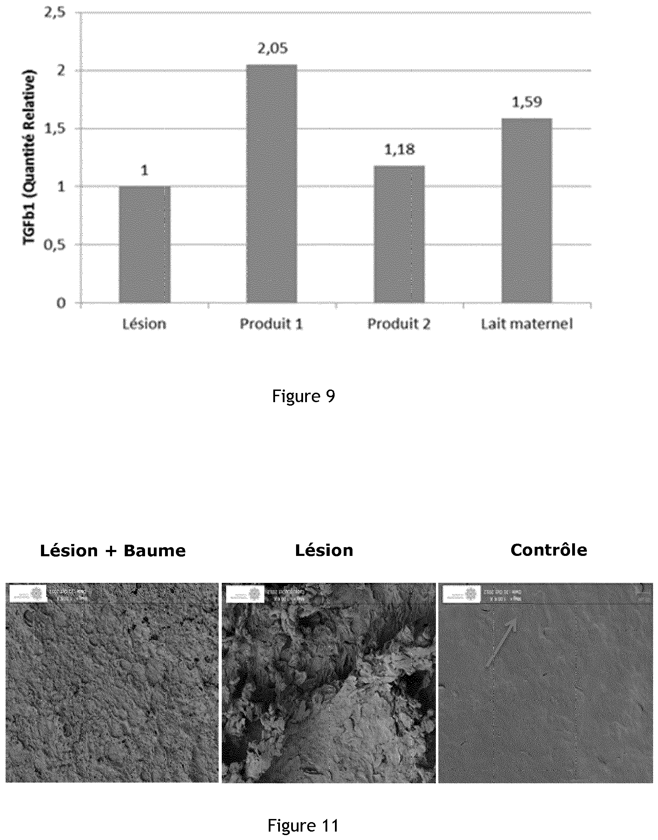

Les résultats sont représentés dans le tableau 6 et illustrés dans la figure 9.

**Tableau 6**

| | TGF β1 | |
|---|---|---|
| | (Expression génique en quantité relative) | |
| Tissu lésé | 1,00 | |
| Tissu lésé + produit 1 | 2,05 | +105% |
| Tissu lésé + produit 2 | 1,18 | +18% |
| Tissu lésé + lait maternel | 1,59 | +59% |

Le TGF β1 est un facteur de croissance qui contrôle de nombreuses fonctions cellulaires, il est notamment impliqué dans la régulation de la cicatrisation en stimulant la ré-épithélisation, phase précoce du processus de cicatrisation épidermique.

Le produit 1 a induit une augmentation importante (+105%) de l'expression du TGF β1 dans les tissus lésés, le produit 2 a entrainé une augmentation faible (+18%) et le lait maternel a induit une augmentation à un niveau intermédiaire (+59%).

Le produit 1 et, dans une moindre mesure, le lait maternel contribuent à initier le processus de cicatrisation pour un effet réparateur.

### d. Evaluation de l'initiation de la cicatrisation : expression de l'intégrine β1

Les résultats sont illustrés dans la figure 10.

Dans les tissus contrôles, les résultats montrent un marquage intense de l'intégrine β1 associé à la membrane plasmique des kératinocytes (flèches jaunes). Cette localisation membranaire de l'intégrine β1 montre la disponibilité/fonctionnalité du récepteur.

La lésion des tissus entraine une forte diminution du marquage de l'intégrine β1, celui ci a pratiquement disparu, ce qui montre une altération du récepteur.

En présence du baume allaitement (produit 1), un marquage membranaire de l'intégrine β1 est récupéré (flèches jaunes), témoin de la restauration de la fonctionnalité du récepteur.

Le baume allaitement (produit 1) restaure la production de l'intégrine β1, permettant ainsi de restaurer les contacts intercellulaires pour initier le processus de réparation.

### 3. Conclusion

Un modèle de mamelon obtenu par reconstruction d'un épiderme à partir d'un lot de kératinocytes surexprimant la filaggrine a été utilisé afin d'évaluer comparativement l'efficacité réparatrice et anti-inflammatoire de produits cosmétiques destinés au soin du mamelon en comparaison avec le lait maternel. L'épiderme reconstruit de « mamelon » a été lésé en surface afin de mimer une lésion.

Sur ce modèle d'épiderme de mamelon lésé, des différences d'efficacité ont été mises en évidence entre les deux produits testés et le lait maternel (connu pour ses propriétés réparatrices) en termes d'action de protection (protection de l'intégrité des membranes cellulaires / LDH), de réparation (initiation de la cicatrisation / TGF β1 ; intégrine β1) et d'apaisement (anti-inflammatoire /TNFα).

Ces résultats confirment l'intérêt du modèle mis en place comme un modèle mimant l'épiderme de mamelon, permettant d'évaluer comparativement différentes formules et permettant l'identification de formules efficaces pour le soin des crevasses/fissures du mamelon.

### E. Etude 3 : Utilisation d'un modèle d'épiderme reconstruit de mamelon pour visualiser l'effet protecteur et réparateur d'un baume allaitement

Les étapes précédentes ont montré l'intérêt du modèle d'épiderme de mamelon mis en place pour l'évaluation de l'activité ou l'identification d'une formulation pour traiter les lésions de la peau du mamelon en se basant sur la mesure de marqueurs d'un effet réparateur, protecteur ou anti-inflammatoire.

Afin de valider les conclusions obtenues par l'analyse du niveau de production des marqueurs, nous avons cherché à visualiser l'effet d'une lésion du modèle d'épiderme de mamelon et l'effet réparateur d'un produit en utilisant la technique de microscopie électronique.

### 1. Matériel et Méthodes

Un épiderme reconstruit, obtenu à partir d'un lot de kératinocytes surexprimant la filaggrine (comme décrit précédemment), a été utilisé comme modèle de mamelon.

Une lésion (blessure mécanique) en surface de l'épiderme de mamelon a été réalisée, afin de mimer une crevasse ou fissure du mamelon.

Un baume allaitement correspondant à la formulation « baume allaitement 3 » a ensuite été appliqué topiquement.

Après incubation pendant 4 heures, les épidermes ont été fixés, déshydratés et recouverts d'une couche d'or avant d'être observés au microscope électronique à balayage (Zeiss).

### 2. Résultats

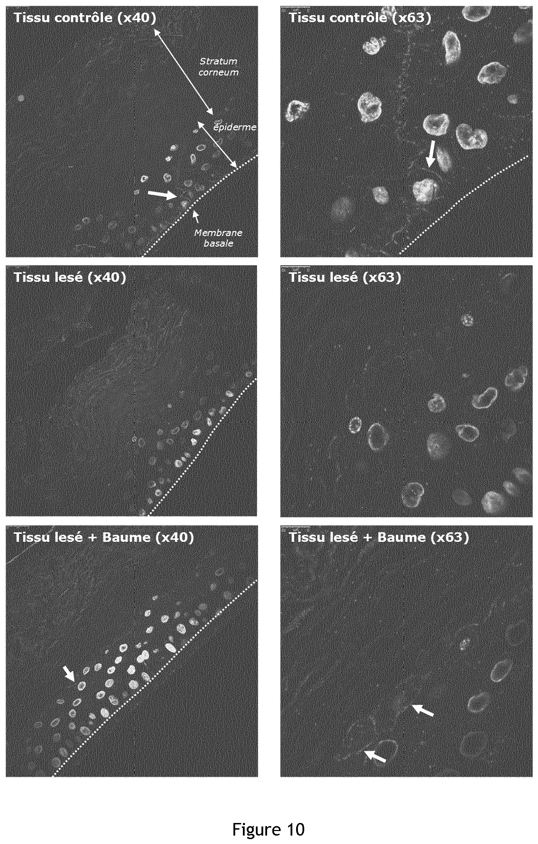

Les résultats sont illustrés dans la figure 11.

Les kératinocytes épidermiques forment une enveloppe cornée au cours des dernières étapes de leur différenciation. Cette enveloppe cornée assure la cohésion et solidité du stratum corneum et joue un rôle primordial dans la fonction barrière de l'épiderme.

L'observation en Microscopie Electronique à Balayage de la surface des tissus reconstruits montre dans les tissus contrôles une surface cutanée intacte, homogène et lisse (qui correspond à l'enveloppe cornée) où l'on distingue le contour des cellules (cornéocytes ; flèche bleue).

La lésion entraine une blessure profonde avec destruction de l'enveloppe cornée.

L'application du baume allaitement après la lésion accélère visiblement la réparation de cette blessure : la surface est plus régulière et plus lisse, montrant la restauration de l'enveloppe cornée.

### 3. Conclusion

Ces observations en Microscopie Electronique à Balayage confirment de façon directe l'effet réparateur et protecteur du baume allaitement mis en évidence par le biais de la production de différents marqueurs lors des étapes précédentes.

Ainsi ces résultats confirment :
- l'intérêt du modèle mis en place comme un modèle mimant la peau ou l'épiderme de mamelon fissuré et permettant d'évaluer ou d'identifier l'effet réparateur ou protecteur d'une formulation,
- le choix des marqueurs (dans les étapes précédentes) et leur validité comme marqueurs représentatifs d'un effet réparateur et protecteur.

## Revendications

1. Modèle de peau de mamelon humain *in vitro* **caractérisé en ce qu'**il comprend des kératinocytes d'origine humaine surexprimant la filaggrine et **en ce qu'**il comprend une lésion mimant une crevasse, dans lequel lesdits kératinocytes présentent de façon constitutive un niveau d'expression du marqueur biologique filaggrine supérieur à un niveau d'expression de référence, ledit niveau d'expression de référence correspondant à un niveau d'expression habituellement observé dans les cultures de kératinocytes primaires d'origine humaine ; et **en ce que** ledit modèle est un modèle d'épiderme comprenant un support matriciel.

2. Procédé d'évaluation de l'activité *in vitro* d'un actif ou d'une formulation sur la cicatrisation de la peau du mamelon et/ou sur la diminution de l'inflammation de la peau du mamelon, **caractérisé en ce que** ledit procédé comprend au moins les étapes suivantes:
a) mettre en contact ledit actif ou ladite formulation avec un modèle de peau selon la revendication 1;
b) mesurer le niveau de production d'au moins un marqueur biologique dans le modèle de peau de mamelon de l'étape a), **caractérisé en ce que** ledit marqueur biologique est choisi parmi le groupe consistant en:
- les marqueurs de l'intégrité épidermique, ledit marqueur de l'intégrité épidermique étant de préférence la lactate déshydrogénase (LDH); et
- les marqueurs de la barrière épidermique, ledit marqueur de la barrière épidermique étant de préférence la filaggrine; et
- les marqueurs de l'inflammation épidermique, ledit marqueur de l'inflammation épidermique étant de préférence choisi parmi les cytokines, en particulier le TNFα; et
- les marqueurs de la cicatrisation, ledit marqueur de la cicatrisation étant de préférence choisi parmi les intégrines, en particulier l'intégrine β1, et le TGF β1;
c) comparer le niveau de production d'au moins un marqueur biologique obtenu à l'étape b) avec un niveau de production de référence ;
d) évaluer l'efficacité dudit actif ou de ladite formulation sur la cicatrisation de la peau du mamelon et/ou sur la diminution de l'inflammation de la peau du mamelon en fonction de la comparaison de l'étape c).

3. Procédé d'identification d'un actif ou d'une formulation permettant de traiter les lésions de la peau du mamelon, en particulier les crevasses, ledit procédé comprenant les étapes suivantes :
a) mettre en contact un actif candidat ou une formulation candidate avec un modèle de peau selon la revendication 1;
b) mesurer le niveau de production d'au moins un marqueur biologique dans le modèle de peau de mamelon de l'étape a), **caractérisé en ce que** ledit marqueur biologique est choisi parmi le groupe consistant en:
- les marqueurs de l'intégrité épidermique, ledit marqueur de l'intégrité épidermique étant de préférence la lactate déshydrogénase (LDH); et
- les marqueurs de la barrière épidermique, ledit marqueur de la barrière épidermique étant de préférence la filaggrine; et
- les marqueurs de l'inflammation épidermique, ledit marqueur de l'inflammation épidermique étant de préférence choisi parmi les cytokines, en particulier le TNFα; et
- les marqueurs de la cicatrisation, ledit marqueur de la cicatrisation étant de préférence choisi parmi les intégrines, en particulier l'intégrine β1, et le TGF β1;
c) comparer le niveau de production d'au moins un marqueur biologique obtenu à l'étape b) avec un niveau de production de référence ;
d) déterminer si ledit actif ou ladite formulation candidate est une formulation permettant de traiter les lésions de la peau du mamelon, en particulier les crevasses, en fonction de la comparaison de l'étape c).

4. Procédé selon l'une des revendications 2 ou 3, **caractérisé en ce que** l'étape b) comprend la mesure du niveau de production d'une combinaison de marqueurs biologiques comprenant ou consistant en :
- au moins un marqueur de l'intégrité épidermique, ledit marqueur de l'intégrité épidermique étant de préférence la lactate déshydrogénase (LDH); et
- au moins un marqueur de la barrière épidermique, ledit marqueur de la barrière épidermique étant de préférence la filaggrine; et
- au moins un marqueur de l'inflammation épidermique, ledit marqueur de l'inflammation épidermique étant de préférence choisi parmi les cytokines, en particulier le TNFα; et
- au moins un marqueur de la cicatrisation, ledit marqueur de la cicatrisation étant de préférence choisi parmi les intégrines, en particulier l'intégrine β1, et le TGF β1.

5. Utilisation d'un modèle de peau selon la revendication 1, pour étudier la peau du mamelon.

## Patentansprüche

1. Menschliches *In-Vitro*-Mamillenhautmodell, **dadurch gekennzeichnet, dass** es Keratinozyten menschlichen Ursprungs umfasst, die Filaggrin überexprimieren, und dadurch, dass es eine Läsion umfasst, die einen Riss nachahmt, wobei die Keratinozyten konstitutiv ein Expressionsniveau des biologischen Markers Filaggrin aufweisen, das höher als ein Bezugsexpressionsniveau ist, wobei das Bezugsexpressionsniveau einem Expressionsniveau entspricht, das für gewöhnlich in den primären Keratinocytkulturen menschlichen Ursprungs beobachtet wird; und dadurch, dass das Modell ein Epidermismodell ist, das einen Matrixträger umfasst.

2. Verfahren zur Bewertung der *In-Vitro*-Aktivität eines Wirkstoffs oder einer Formulierung auf die Heilung der Mamillenhaut und/oder auf die Verminderung der Entzündung der Mamillenhaut, **dadurch gekennzeichnet, dass** das Verfahren mindestens die folgenden Schritte umfasst:
a) Inkontaktbringen des Wirkstoffs oder der Formulierung mit einem Hautmodell nach Anspruch 1;
b) Messen des Niveaus der Produktion von mindestens einem biologischen Marker in dem Mamillenhautmodell von Schritt a), **dadurch gekennzeichnet, dass** der biologische Marker in der Gruppe ausgewählt wird, die besteht aus:
- den Markern für die Integrität der Epidermis, wobei der Marker für die Integrität der Epidermis vorzugsweise Lactatdehydrogenase (LDH) ist; und
- den Markern für die epidermale Barriere, wobei der Marker für die epidermale Barriere vorzugsweise Filaggrin ist; und
- den Markern für die Hautentzündung, wobei der Marker für die Hautentzündung vorzugsweise unter Zytokinen, insbesondere TNFα, ausgewählt wird; und
- den Heilungsmarkern, wobei der Heilungsmarker vorzugsweise unter den Integrinen, insbesondere dem Integrin β1, und TGF β1, ausgewählt wird;
c) Vergleichen des Niveaus der Produktion von mindestens einem in Schritt b) erhaltenen biologischen Marker mit einem Bezugsproduktionsniveau;
d) Bewerten der Wirksamkeit des Wirkstoffs oder der Formulierung auf die Heilung der Mamillenhaut und/oder auf die Verminderung der Entzündung der Mamillenhaut in Abhängigkeit von dem Vergleich von Schritt c).

3. Verfahren zur Identifikation eines Wirkstoffs oder einer Formulierung, die das Behandeln von Läsionen der Mamillenhaut, insbesondere von Rissen, umfasst, wobei das Verfahren die folgenden Schritte umfasst:
a) Inkontaktbringen eines Kandidatenwirkstoffs oder einer Kandidatenformulierung mit einem Hautmodell nach Anspruch 1;
b) Messen des Niveaus der Produktion von mindestens einem biologischen Marker in dem Mamillenhautmodell von Schritt a), **dadurch gekennzeichnet, dass** der biologische Marker in der Gruppe ausgewählt wird, die besteht aus:
- den Markern für die Integrität der Epidermis, wobei der Marker für die Integrität der Epidermis vorzugsweise Lactatdehydrogenase (LDH) ist; und
- den Markern für die epidermale Barriere, wobei der Marker für die epidermale Barriere vorzugsweise Filaggrin ist; und
- den Markern für die Hautentzündung, wobei der Marker für die Hautentzündung vorzugsweise unter Zytokinen, insbesondere TNFα, ausgewählt wird; und
- den Heilungsmarkern, wobei der Heilungsmarker vorzugsweise unter den Integrinen, insbesondere dem Integrin β1, und TGF β1, ausgewählt wird;
c) Vergleichen des Niveaus der Produktion von mindestens einem in Schritt b) erhaltenen biologischen Marker mit einem Bezugsproduktionsniveau;
d) Bestimmen, ob der/die Kandidatenwirkstoff oder -formulierung eine Formulierung ist, die die Behandlung von Läsionen der Mamillenhaut, insbesondere von Rissen, ermöglicht, in Abhängigkeit von dem Vergleich von Schritt c).

4. Verfahren nach einem der Ansprüche 2 oder 3, **dadurch gekennzeichnet, dass** der Schritt b) das Messen des Niveaus der Produktion einer Kombination von biologischen Markern umfasst, die umfassen oder bestehen aus:
- mindestens einem Marker für die Integrität der Epidermis, wobei der Marker für die Integrität der Epidermis vorzugsweise Lactatdehydrogenase (LDH) ist; und
- mindestens einem Marker für die epidermale Barriere, wobei der Marker für die epidermale Barriere vorzugsweise Filaggrin ist; und
- mindestens einem Marker für die Hautentzündung, wobei der Marker für die Hautentzündung vorzugsweise unter Zytokinen, insbesondere TNFα, ausgewählt wird; und
- mindestens einem Heilungsmarker, wobei der Heilungsmarker vorzugsweise unter den Integrinen, insbesondere dem Integrin β1, und TGF β1, ausgewählt wird.

5. Verwendung eines Modells nach Anspruch 1 zur Untersuchung der Mamillenhaut.

## Claims

1. *In-vitro* Human nipple skin model **characterized in that** it comprises keratinocytes of human origin overexpressing filaggrin and **in that** it comprises a lesion mimicking a crack, in which said keratinocytes constitutively exhibit a level of expression of the filaggrin biological marker greater than a reference level of expression, said reference level of expression corresponding to a level of expression usually observed in primary cultures of keratinocytes of human origin; and **in that** said model is an epidermis model comprising a matrix support.

2. Method for evaluating the *in-vitro* activity of an active ingredient or formulation on nipple skin healing and/or reduction of nipple skin inflammation, **characterized in that** said method comprises at least the following steps:
a) contacting said active ingredient or said formulation with a skin model according to claim 1;
b) measuring the production level of at least one biological marker in the nipple skin model of step a), **characterized in that** said biological marker is selected from among the group consisting of:
- epidermal integrity markers, said epidermal integrity marker being preferably lactate dehydrogenase (LDH); and
- epidermal barrier markers, said epidermal barrier marker being preferably filaggrin; and
- epidermal inflammation markers, said epidermal inflammation marker being preferably selected from among cytokines, in particularly TNFα; and
- healing markers, said healing marker being preferably selected from among integrins, in particular integrin β1, and TGF β1;
c) comparing the production level of at least one biological marker obtained at step b) with a reference production level;
d) evaluating the efficacy of said active ingredient or said formulation on nipple skin healing and/or reduction of nipple skin inflammation depending on the comparison of step c).

3. Method for identifying an active ingredient or a formulation for treating nipple skin lesions, in particular cracks, said method comprising the following steps:
a) contacting a candidate active ingredient or a candidate formulation with a skin model according to claim 1;
b) measuring the production level of at least one biological marker in the nipple skin model of step a), **characterized in that** said biological marker is selected from among the group consisting of:
- epidermal integrity markers, said epidermal integrity marker being preferably lactate dehydrogenase (LDH); and
- epidermal barrier markers, said epidermal barrier marker being preferably filaggrin; and
- epidermal inflammation markers, said epidermal inflammation marker being preferably selected from among cytokines, in particular TNFα; and
- healing markers, said healing marker preferably being selected from among the integrins, in particular integrin β1 and TGF β1;
c) comparing the production level of at least one biological marker obtained at step b) with a reference production level;
d) determining if said candidate active ingredient or said candidate formulation is a formulation to treat nipple skin lesions, in particular cracks, according to the comparison of step c).

4. Method according to one of claims 2 or 3, **characterized in that** step b) comprises measuring the production level of a combination of biological markers comprising or consisting of:
- at least one epidermal integrity marker, said epidermal integrity marker being preferably lactate dehydrogenase (LDH); and
- at least one epidermal barrier marker, said epidermal barrier marker being preferably filaggrin; and
- at least one epidermal inflammation marker, said epidermal inflammation marker being preferably selected from among cytokines, in particularly TNFα; and
- at least one healing marker, said healing marker being preferably selected from among integrins, in particular integrin β1, and TGF β1;

5. Use of a skin model according to claims 1, to study nipple skin.
